# EUROPÄISCHE PATENTANMELDUNG

(11) **EP 4 279 143 A1**
(43) Veröffentlichungstag der Anmeldung: **22.11.2023**
(21) Anmeldenummer: 23172802.3
(22) Anmeldetag: 11.05.2023
(51) Int. Cl.: A62B 7/10, A62B 18/02, A62B 18/08, A62B 23/02

(54) **ATEMMASKE MIT PARTIKELFILTER**

(30) Priorität: 17.05.2022 DE 102022112288
(71) Anmelder: Löwenstein Medical Technology S.A., 2557 Luxembourg (LU)
(72) Erfinder: EIFLER, Martin, 25348 Glückstadt (DE)
(74) Vertreter: Marx, Thomas

(57) **Zusammenfassung**

Die Erfindung betrifft eine Atemmaske 1 umfassend einen Maskenkörper 10, einen Maskenwulst 12, zumindest einen Maskenflügel 14 und zumindest ein Filterelement 40, wobei der Maskenwulst 12 mit dem Maskenkörper 10 verbunden ist, wobei der Maskenflügel 14 mit dem Maskenkörper 10 und/oder dem Maskenwulst 12 verbunden ist, wobei der Maskenwulst 12 zumindest abschnittsweise zur Anlage an der Haut eines Anwenders ausgebildet ist, wobei der Maskenwulst 12 bei Anwendung der Atemmaske 1 derart an der Haut des Anwenders anliegt, dass die Atemmaske 1 im Wesentlichen atemgasdicht abschließt, wobei der Maskenwulst 12 eine Aufnahmeöffnung 32 aufweist, die ausgebildet und eingerichtet ist, bei Anwendung der Atemmaske 1 zumindest Nase und Mund des Anwenders aufzunehmen, dadurch gekennzeichnet dass das Filterelement 40 mit dem Maskenflügel 14 verbunden ist, wobei das Filterelement 40 derart eingerichtet und ausgebildet ist, dass es zumindest bereichsweise von Atemgas durchströmbar ist.

## Beschreibung

Partikelfiltrierende Halbmasken werden als Atemschutz gegen Aerosole aus festen oder flüssigen, nicht leicht flüchtigen Partikeln eingesetzt. Sie sind nach der europäischen Norm DIN EN 149 geprüft und erfüllen die Anforderungen dieser Norm. Die Norm unterscheidet je nach Rückhaltevermögen des Partikelfilters die Geräteklassen FFP1, FFP2 und FFP3. Üblicherweise besteht die Maske vollständig aus dem nicht auswechselbaren Filtermaterial. Beispiele solcher Masken werden in der DE 10 2014 221 311 B3 und der DE 20 2013 011 420 U1 offenbart.

Davon zu unterscheiden sind "Halbmasken oder Vollmasken mit Partikelfilter", welche eine oder mehrere Anschlussmöglichkeiten für auswechselbare Partikelfilter besitzen. Halbmasken oder Vollmasken mit auswechselbarem Partikelfilter gemäß der DE 10 2014 001937 B3 oder der DE 20 2014 001 315 U1 sind derzeit recht klobige Masken, welche insbesondere im Industriebereich eingesetzt werden und das Gesichtsfeld stark einschränken. Zudem weisen solche Masken zumindest ein Ventil für die Einatmung und/oder Ausatmung auf.

Entscheidend für die Schutzwirkung einer Atemschutzmaske ist die Dichtigkeit. Diese ergibt sich aus dem Filterdurchlass und der so genannten Verpassungsleckage, die durch Undichtigkeiten zwischen der Dichtlinie der Maske und dem Gesicht des Trägers entsteht. Die Schutzwirkung nimmt von einer FFP1-Maske (Gesamtleckage max. 22 %) über eine FFP2-Maske (Gesamtleckage max. 8 %) bis zur FFP3-Maske (Gesamtleckage max. 2 %) zu. Mit der Zunahme der Schutzwirkung steigt auch der Atemwiderstand der Maske. Durch ein Ausatemventil wird der Ausatemwiderstand herabgesetzt. Damit ist die partikelfiltrierende Halbmaske für den Träger weniger belastend und deshalb bevorzugt einzusetzen.

Medizinische Atemmasken, die zum Infektionsschutz eingesetzt werden, benötigen ebenfalls partikelfiltrierende Eigenschaften, um einen Schutz vor infektiösen Partikeln und/oder Aerosolen zu bieten. Viren, Bakterien und anderes infektiöses Material kann an kleinste Tröpfchen gebunden sein und damit im Filter aufgefangen werden. Infektionsschutzmasken können sowohl für den Träger als auch für andere Personen einen Schutz gegen Bakterien und Viren bieten.

Atemmasken nach dem Stand der Technik weisen folgende wesentliche Nachteile auf:
- Große Menge an Abfall bei Einwegmasken
- Geringe Anpassbarkeit und hohe Leckage bei Einheitsgrößen bzw. Einheitsformen
- Klobiger Aufbau
- Atemmasken mit Ausatemventilen können den Anwender schützen jedoch nicht andere Personen, da infektiöses Material über das Ausatemventil ungefiltert in die Umgebung abgegeben wird
- Kostenintensive Herstellung
- Komplizierte Handhabung, insbesondere beim Filterwechsel

Es ist Aufgabe der vorliegenden Erfindung, eine Atemmaske bereitzustellen, die an alle Gesichtsformen anpassbar ist sowie einfach zu handhaben ist und dennoch einen sicheren Schutz bietet und den Anwender nicht beeinträchtigt.

Diese Aufgabe wird gelöst mit einer Atemmaske des Anspruchs 1 sowie mit einem Filterelement gemäß Anspruch 55. Weiterbildungen und vorteilhafte Ausgestaltungen sind Gegenstand der Unteransprüche. Weitere Vorteile und Merkmale ergeben sich aus der allgemeinen Beschreibung und der Beschreibung der Ausführungsbeispiele.

Die Erfindung betrifft eine Atemmaske umfassend einen Maskenkörper, einen Maskenwulst, zumindest einen Maskenflügel und zumindest ein Filterelement, wobei der Maskenwulst mit dem Maskenkörper verbunden ist, wobei der Maskenflügel mit dem Maskenkörper und/oder dem Maskenwulst verbunden ist, wobei der Maskenwulst zumindest abschnittsweise zur Anlage an der Haut eines Anwenders ausgebildet ist, wobei der Maskenwulst bei Anwendung der Atemmaske derart an der Haut des Anwenders anliegt, dass die Atemmaske im Wesentlichen atemgasdicht abschließt, wobei der Maskenwulst eine Aufnahmeöffnung aufweist, die ausgebildet und eingerichtet ist, bei Anwendung der Atemmaske zumindest Nase und Mund des Anwenders aufzunehmen dadurch gekennzeichnet, dass das Filterelement mit dem Maskenflügel verbunden ist, wobei das Filterelement derart eingerichtet und ausgebildet ist, dass es zumindest bereichsweise von Atemgas durchströmbar ist.

In manchen Ausführungsformen ist die Atemmaske dadurch gekennzeichnet, dass die Atemmaske mindestens zwei Maskenflügel umfasst, die mit jeweils mindestens einem Filterelement verbunden sind. In manchen Ausführungsformen ist die Atemmaske dadurch gekennzeichnet, dass die Verbindung von Maskenflügeln und Filterelementen reversibel oder irreversibel ist.

In manchen Ausführungsformen ist die Atemmaske dadurch gekennzeichnet, dass die Atemmaske sich in einem Anwendungszustand befindet, wenn die Filterelemente mit den Maskenflügeln verbunden sind.

In manchen Ausführungsformen ist die Atemmaske dadurch gekennzeichnet, dass das Filterelement ein Filtermaterial ist und/oder ein Filtermaterial umfasst.

In manchen Ausführungsformen ist die Atemmaske dadurch gekennzeichnet, dass das Filtermaterial anorganische Partikel und/oder organische Partikel, insbesondere Keime aus dem Atemgas filtert und zurückhält. In manchen Ausführungsformen ist die Atemmaske dadurch gekennzeichnet, dass das Filtermaterial aus Kunststofffasern gefertigt ist, beispielsweise aus Polypropylen (PP) und/oder Polytetrafluorethylen (PTFE).

In manchen Ausführungsformen ist die Atemmaske dadurch gekennzeichnet, dass das Filtermaterial eine Fläche von mindestens 5 cm² aufweist, bevorzugt mindestens 20 cm², insbesondere mindestens 35 cm².

In manchen Ausführungsformen ist die Atemmaske dadurch gekennzeichnet, dass das Filtermaterial eine Fläche von mindestens 5 cm² aufweist, wobei die Fläche des Filtermaterials bevorzugt in einem Bereich zwischen 35 cm² und 55 cm² liegt.

In manchen Ausführungsformen ist die Atemmaske dadurch gekennzeichnet, dass das Filtermaterial austauschbar ist.

In manchen Ausführungsformen ist die Atemmaske dadurch gekennzeichnet, dass der Maskenkörper, der Maskenwulst und die Maskenflügel mit den Filterelementen bei Anwendung der Atemmaske einen Atemgasraum definieren, in dem sich Mund und Nase des Anwenders befinden, wobei eine Atemgasströmung vom Mund und Nase des Anwenders weg sowie zum Mund und Nase des Anwenders hin ausschließlich durch das Filtermaterial möglich ist.

In manchen Ausführungsformen ist die Atemmaske dadurch gekennzeichnet, dass der Atemgasraum ein Volumen aufweist, das derart gering ist, dass eine CO2-Auswaschung aus der Atemluft des Anwenders erfolgt. In manchen Ausführungsformen ist die Atemmaske dadurch gekennzeichnet, dass der Atemgasraum ein Volumen von maximal 500 ml, bevorzugt maximal 200 ml, insbesondere maximal 120 ml aufweist.

In manchen Ausführungsformen ist die Atemmaske dadurch gekennzeichnet, dass die Atemmaske aus einem elastomeren und/oder einem formstabilen Kunststoff gefertigt ist. In manchen Ausführungsformen ist die Atemmaske dadurch gekennzeichnet, dass die Atemmaske aus einem Kunststoff gefertigt ist ausgewählt aus der Gruppe Polycarbonate (PC), Polystyrole (PS), Polyamide (PA), Polypropylene (PP), Polyoxymethylene (POM), Polyacrylnitrile (PAN), Thermoplastische Elastomere (TPE), Acrylnitril-Styrol-Acrylester (ASA), Acrylnitril-Butadien-Styrole (ABS), Styrol-Acrylnitrile (SAN), Styrol-Methyl-Methacrylate (SMMA), Polyethylene (PE), Cycloolefin-Copolymere (COC), Polytetrafluorethylene (PTFE), Silikone.

In manchen Ausführungsformen ist die Atemmaske dadurch gekennzeichnet, dass der Maskenkörper und/oder der Maskenwulst und/oder die Maskenflügel mehrstückig oder einstückig gefertigt sind, bevorzugt einstückig. In manchen Ausführungsformen ist die Atemmaske dadurch gekennzeichnet, dass zumindest der Maskenkörper und die Maskenflügel einstückig gefertigt sind und die Filterelemente reversibel mit den Maskenflügeln verbunden sind.

In manchen Ausführungsformen ist die Atemmaske dadurch gekennzeichnet, dass die Filterelemente aus einem formstabilen Kunststoff gefertigt sind, beispielsweise aus Polypropylen.

In manchen Ausführungsformen ist die Atemmaske dadurch gekennzeichnet, dass der Maskenkörper, der Maskenwulst und die Maskenflügel im Wesentlichen aus einem elastomeren Kunststoff gefertigt sind. In manchen Ausführungsformen ist die Atemmaske dadurch gekennzeichnet, dass der Maskenkörper, der Maskenwulst und die Maskenflügel im Wesentlichen aus einem Silikon gefertigt sind. In manchen Ausführungsformen ist die Atemmaske dadurch gekennzeichnet, dass das Silikon klarsichtig ist, wobei das Silikon eine Lichtdurchlässigkeit von mindestens 50% aufweist, bevorzugt von mindestens 80%, besonders bevorzugt von mindestens 90%.

In manchen Ausführungsformen ist die Atemmaske dadurch gekennzeichnet, dass der Maskenkörper und/oder der Maskenwulst und/oder die Maskenflügel eine Shore-Härte von 30 bis 60 Shore A aufweisen. In manchen Ausführungsformen ist die Atemmaske dadurch gekennzeichnet, dass der Maskenkörper und/oder der Maskenwulst und/oder die Maskenflügel eine maximale Dehnbarkeit zwischen 100% und 800% aufweisen.

In manchen Ausführungsformen ist die Atemmaske dadurch gekennzeichnet, dass in und/oder an das Material des Maskenkörpers weitere Materialien zur Verstärkung und/oder Versteifung eingebracht und/oder aufgebracht sind.

In manchen Ausführungsformen ist die Atemmaske dadurch gekennzeichnet, dass die Atemmaske desinfizierbar und/oder sterilisierbar ist. In manchen Ausführungsformen ist die Atemmaske dadurch gekennzeichnet, dass die Atemmaske autoklavierbar ist. In manchen Ausführungsformen ist die Atemmaske dadurch gekennzeichnet, dass zumindest der Maskenkörper, der Maskenwulst und die Maskenflügel autoklavierbar sind.

In manchen Ausführungsformen ist die Atemmaske dadurch gekennzeichnet, dass die Atemmaske mindestens eine Bänderungshalterung umfasst, die eingerichtet ist, zumindest ein Kopfbefestigungsband aufzunehmen. In manchen Ausführungsformen ist die Atemmaske dadurch gekennzeichnet, dass die Bänderungshalterungen als Vorsprung ausgebildet sind und mindestens eine Durchführungsöffnung zur Durchführung des Kopfbefestigungsbandes enthalten. In manchen Ausführungsformen ist die Atemmaske dadurch gekennzeichnet, dass die Bänderungshalterungen an den Maskenflügeln und/oder an den Filterelementen angeordnet sind. In manchen Ausführungsformen ist die Atemmaske dadurch gekennzeichnet, dass die Bänderungshalterungen an den Filterelementen angeordnet sind.

In manchen Ausführungsformen ist die Atemmaske dadurch gekennzeichnet, dass die Maskenflügel mindestens einen Flügelkanal mit einem Lumen umfassen.

In manchen Ausführungsformen ist die Atemmaske dadurch gekennzeichnet, dass die Maskenflügel mindestens eine Aufnahmeeinrichtung zur Aufnahme des Filterelementes umfassen, wobei die Aufnahmeeinrichtung weitestgehend formkomplementär zum Filterelement ausgebildet ist.

In manchen Ausführungsformen ist die Atemmaske dadurch gekennzeichnet, dass die Aufnahmeeinrichtung und das Filterelement im Wesentlichen eine mehreckige Grundform aufweisen, bevorzugt eine viereckige Grundform. In manchen Ausführungsformen ist die Atemmaske dadurch gekennzeichnet, dass die Grundform von Aufnahmeeinrichtung und Filterelement im Wesentlichen rautenförmig oder trapezförmig ist, bevorzugt trapezförmig.

In manchen Ausführungsformen ist die Atemmaske dadurch gekennzeichnet, dass die Aufnahmeeinrichtung mindestens eine Aufnahme-Seitenwand umfasst, die elastisch verformbar ist. In manchen Ausführungsformen ist die Atemmaske dadurch gekennzeichnet, dass das Filterelement nach Aufnahme in die Aufnahmeeinrichtung von der Aufnahme-Seitenwand zumindest teilweise umgeben ist und durch diese in der Aufnahmeeinrichtung fixiert wird.

In manchen Ausführungsformen ist die Atemmaske dadurch gekennzeichnet, dass das Filterelement ein Vorderteil und ein Hinterteil und das Filtermaterial umfasst, wobei das Filtermaterial zwischen dem Vorderteil und dem Hinterteil angeordnet ist. In manchen Ausführungsformen ist die Atemmaske dadurch gekennzeichnet, dass das Vorderteil und das Hinterteil miteinander zumindest teilweise irreversibel verbunden sind oder reversibel miteinander verbunden sind.

In manchen Ausführungsformen ist die Atemmaske dadurch gekennzeichnet, dass das Filtermaterial zwischen dem Vorderteil und dem Hinterteil austauschbar angeordnet ist.

In manchen Ausführungsformen ist die Atemmaske dadurch gekennzeichnet, dass das Filterelement mindestens einen Kanal mit einem Lumen umfasst. In manchen Ausführungsformen ist die Atemmaske dadurch gekennzeichnet, dass das Hinterteil eine Rückwand und eine Seitenwand umfasst, die zumindest bereichsweise den Kanal begrenzen.

In manchen Ausführungsformen ist die Atemmaske dadurch gekennzeichnet, dass das Hinterteil mindestens eine Kanalwand mit einer Basis und einer Stirnseite umfasst, wobei die Kanalwand den Kanal in Untereinheiten unterteilt.

In manchen Ausführungsformen ist die Atemmaske dadurch gekennzeichnet, dass das Hinterteil eine Auflagefläche umfasst, wobei das Filtermaterial derart auf der Auflagefläche und/oder auf den Stirnseiten der Kanalwände platziert wird, dass der Kanal zumindest bereichsweise durch das Filtermaterial begrenzt wird.

In manchen Ausführungsformen ist die Atemmaske dadurch gekennzeichnet, dass die Maskenflügel mindestens einen Flügelkanal mit einem Lumen umfassen und dass das Filterelement mindestens einen Kanal mit einem Lumen umfasst und dass das Filterelement ein Tunnelelement mit einem Lumen aufweist, wobei das Tunnelelement zumindest teilweise in dem Flügelkanal der Maskenflügel angeordnet ist, wobei die Lumina zumindest teilweise den Atemgasraum definieren.

In manchen Ausführungsformen ist die Atemmaske dadurch gekennzeichnet, dass das Filterelement ein Vorderteil und ein Hinterteil mit einer Auflagefläche und das Filtermaterial umfasst, wobei das Filtermaterial zwischen dem Vorderteil und dem Hinterteil angeordnet ist, wobei das Filtermaterial derart auf der Auflagefläche platziert ist, dass der Kanal zumindest bereichsweise durch das Filtermaterial begrenzt ist.

In manchen Ausführungsformen ist die Atemmaske dadurch gekennzeichnet, dass das Vorderteil eine Öffnung umfasst, durch die ein Luftaustausch zwischen dem Atemgasraum und der Umgebungsluft stattfindet. In manchen Ausführungsformen ist die Atemmaske dadurch gekennzeichnet, dass das Filtermaterial derart zwischen dem Atemgasraum und der Öffnung platziert ist, dass der Luftaustausch ausschließlich durch das Filtermaterial stattfindet.

In manchen Ausführungsformen ist die Atemmaske dadurch gekennzeichnet, dass das Filterelement ein Tunnelelement mit einem Lumen aufweist. In manchen Ausführungsformen ist die Atemmaske dadurch gekennzeichnet, dass das Hinterteil das Tunnelelement umfasst. In manchen Ausführungsformen ist die Atemmaske dadurch gekennzeichnet, dass das Tunnelelement in einem Anwendungszustand der Atemmaske zumindest teilweise in dem Flügelkanal der Maskenflügel angeordnet ist.

In manchen Ausführungsformen ist die Atemmaske dadurch gekennzeichnet, dass das Tunnelelement mindestens einen Arretierungsvorsprung aufweist, über den das Filterelement in den Maskenflügeln arretiert wird. In manchen Ausführungsformen ist die Atemmaske dadurch gekennzeichnet, dass das Hinterteil und/oder das Vorderteil mindestens eine Arretierungsschiene aufweist, über die das Filterelement in den Maskenflügeln arretiert wird.

In manchen Ausführungsformen ist die Atemmaske dadurch gekennzeichnet, dass der Flügelkanal, das Tunnelelement und der Kanal gasleitend miteinander verbunden sind.

In manchen Ausführungsformen ist die Atemmaske dadurch gekennzeichnet, dass die Lumina zumindest teilweise den Atemgasraum definieren, wobei die Atemmaske derart eingerichtet und ausgebildet ist, dass eine weitgehend störungsfreie Strömung durch den Atemgasraum und das Filtermaterial ermöglicht ist.

In manchen Ausführungsformen ist die Atemmaske dadurch gekennzeichnet, dass das Atemgas vom Mund und/oder Nase des Anwenders über das Lumen des Flügelkanals in das Lumen des Tunnelelements und vom Lumen des Tunnelelements in das Lumen des Kanals und vom Lumen des Kanals ausschließlich durch das Filtermaterial strömt und vice versa.

In manchen Ausführungsformen ist die Atemmaske dadurch gekennzeichnet, dass der Flügelkanal und/oder das Tunnelelement einen hydraulischen Durchmesser von mindestens 10 mm aufweisen, bevorzugt mindestens 12 mm, besonders bevorzugt von mindestens 15 mm.

Die Erfindung betrifft zudem ein Filterelement gemäß Anspruch 55 für eine erfindungsgemäße Atemmaske.

In den Figuren sind Ausführungsbeispiele der erfindungsgemäßen Atemmaske und des erfindungsgemäßen Filterelements dargestellt. Es zeigen:
**Fig. 1** eine Darstellung einer Atemmaske von vorne, wobei Fig. 1A die Atemmaske mit Filterelementen und Fig. 1B die Atemmaske ohne Filterelemente zeigt.
**Fig. 2** eine perspektivische Darstellung einer Atemmaske, wobei Fig. 2A die Atemmaske mit Filterelementen und Fig. 2B die Atemmaske ohne Filterelemente zeigt.
**Fig. 3** eine Darstellung einer Atemmaske mit Filterelementen von hinten.
**Figur 4 A-C** Darstellungen eines beispielhaften Maskenwulstes der Atemmaske aus unterschiedlichen Perspektiven.
**Fig. 5A** eine Darstellung einer Atemmaske mit Filterelementen von oben und **Fig. 5B** eine Atemmaske mit Filterelementen von unten.
**Fig. 6** eine Darstellung einer Atemmaske mit Filterelementen von der Seite.
**Fig. 7** eine vereinfachte Ansicht einer Atemmaske im Querschnitt bei Anlage am Kopf eines Anwenders.
**Fig. 8** eine Explosionsansicht eines Filterelements.
**Fig. 9 A-E** ein Hinterteil eines Filterelements aus unterschiedlichen Perspektiven.
**Fig. 10 A-E** ein Vorderteil eines Filterelements aus unterschiedlichen Perspektiven.
**Fig. 11 A-E** ein Filterelement in einem Anwendungszustand aus unterschiedlichen Perspektiven.
**Fig. 12** eine perspektivische Darstellung einer Atemmaske mit Filterelementen im Querschnitt und **Fig. 12** **A** eine Detailansicht aus Fig. 12.
**Fig. 13** eine Darstellung einer Atemmaske im Querschnitt.

Die in manchen Figuren dargestellten Koordinatensysteme dienen der Verdeutlichung der Ausrichtung der Ansichten. Dabei beschreiben die x-, y- und z-Achsen - soweit nicht anders beschrieben - in jeder Figur die gleiche Richtung. Eine mit einer x-Achse bezeichnete Richtung einer Figur entspricht also in der Regel der Richtung der x-Achse der anderen Figuren.

In den nachfolgenden Ausführungsbeispielen ist eine erfindungsgemäße Atemmaske 1 beschrieben. Weitere Merkmale und Vorteile der vorliegenden Erfindung werden in den nachfolgenden Beschreibungen von Ausführungsbeispielen anhand der Figuren deutlich. Die Erfindung ist nicht auf die dargestellten Ausführungsbeispiele beschränkt.

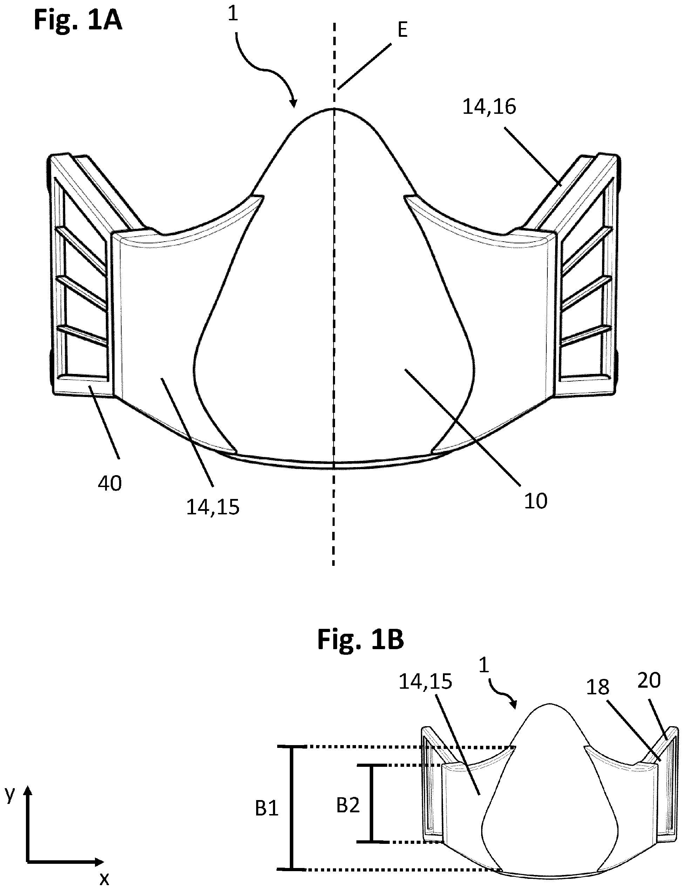

**Fig. 1** zeigt eine Darstellung einer Atemmaske 1 von vorne, wobei Fig. 1A die Atemmaske 1 mit Filterelementen 40 und Fig. 1B die Atemmaske 1 ohne Filterelemente 40 zeigt. Aus Fig. 1 wird ersichtlich, dass die Atemmaske 1 im Wesentlichen spiegelsymmetrisch über eine Symmetrieebene E ausgebildet ist. Über die Symmetrieebene E kann eine linke Hälfte und eine rechte Hälfte definiert werden, die bevorzugt symmetrisch ausgebildet sein können.

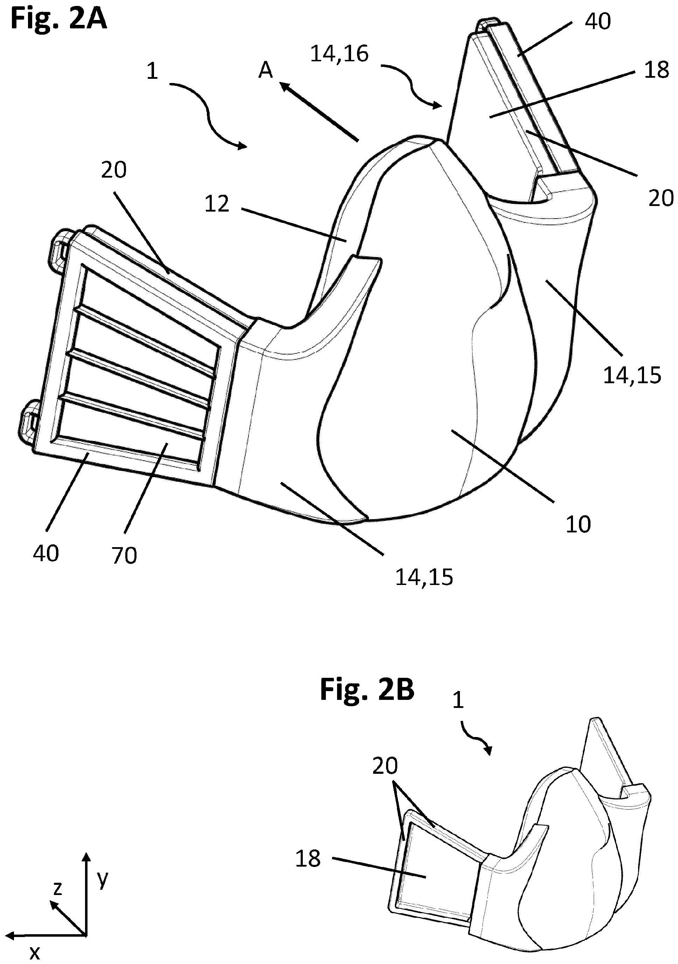

**Fig. 2** zeigt eine perspektivische Darstellung einer Atemmaske 1, wobei Fig. 2A die Atemmaske 1 mit Filterelementen 40 und Fig. 2B die Atemmaske 1 ohne Filterelemente 40 zeigt. Die Atemmaske 1 umfasst einen Maskenkörper 10, einen Maskenwulst 12 und zumindest einen Maskenflügel 14. Die Maskenflügel 14 umfassen jeweils mindestens eine Aufnahmeeinrichtung 16 und mindestens einen Flügelkanal 15. Die Atemmaske 1 umfasst zudem mindestens ein Filterelement 40, das über die Aufnahmeeinrichtung 16 der Maskenflügel 14 mit der Atemmaske 1 reversibel und/oder irreversibel verbunden werden kann. Bevorzugt wird das Filterelement 40 reversibel mit der Atemmaske 1 verbunden.

In einer beispielhaften Ausführungsform gemäß den gezeigten Figuren umfasst die Atemmaske 1 zwei Maskenflügel 14 mit jeweils einer Aufnahmeeinrichtung 16, die jeweils ein Filterelement 40 aufnehmen können. In manchen Ausführungsformen ist es auch denkbar, dass die Atemmaske 1 mehr als zwei Maskenflügel 14 aufweist, beispielsweise vier oder mehr (nicht gezeigt).

Die Atemmaske 1 befindet sich in einem Anwendungszustand, wenn das mindestens eine Filterelement 40 mit der Atemmaske 1 verbunden ist. Sodann kann die Atemmaske 1 von einem Anwender am Kopf 90 angelegt werden (nicht gezeigt) und der Anwender kann atmen. Bei der Atmung mit angelegter Atemmaske 1 wird die Ein- und Ausatemluft im Wesentlichen durch die Filterelemente 40 geleitet. Durch den in Fig. 2A mit A bezeichneten Pfeil wird die generelle Richtung zum Anwender angegeben. Bei Anwendung zeigt der Maskenwulst 12 somit in die Richtung zum Anwender.

Das Filterelement 40 kann in einer einfachen Ausführungsform ein Filtermaterial 70 sein. In einer bevorzugten Ausführungsform kann das Filterelement 40 ein Filtermaterial 70 umfassen. In einem konkreten Ausführungsbeispiel gemäß der Figuren kann in das Filterelement 40 zur Herstellung eines partikelfiltrierenden Anwendungszustandes ein partikelfiltrierendes Filtermaterial 70 eingebracht werden. Das Material des Filtermaterials 70 ist so zu wählen, dass organische und/oder anorganische Partikel durch das Filtermaterial 70 aus dem Atemgas bzw. der Atemluft gefiltert und zurückgehalten werden. Das Filtermaterial 70 ist eingerichtet und ausgebildet, Keime aus dem Atemgas bzw. der Atemluft zu filtern und zurückzuhalten. Keime umfassen im Sinne der Erfindung insbesondere Mikroorganismen und/oder Kleinstlebewesen wie Viren, Viroide, Bakterien, Prionen, Protisten, Algen, Parasiten oder Pilze, sowie deren Derivate oder Vorstufen wie beispielsweise Pilzsporen, Sporen, Allergene, Toxine und dergleichen. Unter Keimen können insbesondere Krankheitserreger und/oder Pathogene verstanden werden.

Beispielsweise besteht das Filtermaterial 70 aus Polypropylen (PP) und/oder Polytetrafluorethylen (PTFE). Beispielsweise sind aber auch andere Materialien zur Verwendung als Filtermaterial 70 denkbar, wie zum Beispiel andere feinmaschig angeordnete Kunststofffasern. Das Filtermaterial 70 kann als Einwegfilter ausgebildet sein. Es ist auch denkbar, dass das Filtermaterial 70 nach der Anwendung wiederaufbereitet werden kann und mehrmals verwendet werden kann.

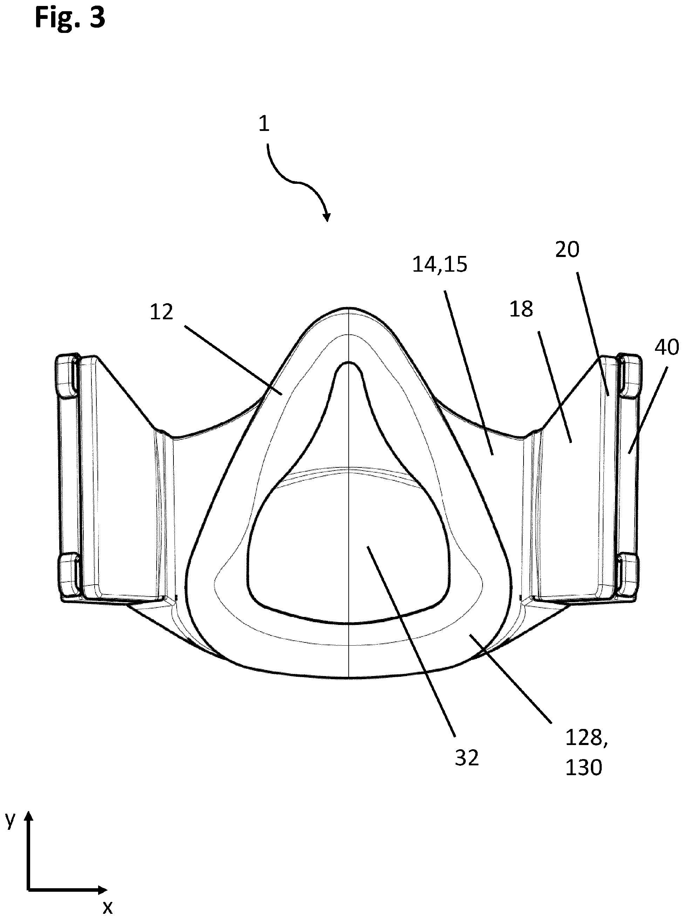

**Fig. 3** zeigt eine Darstellung einer Atemmaske 1 mit Filterelementen 40 von hinten. Fig. 3 zeigt den Maskenwulst 12. Der Maskenwulst 12 weist einen als Dichtlippe 130 ausgeführten Auflagebereich 128 auf und ist derart eingerichtet, dass die Atemmaske 1 bei Anwendung mit der Dichtlippe 130 zumindest abschnittsweise an der Gesichtshaut anliegt und im Wesentlichen atemgasdicht abschließt. Der Maskenwulst 12 begrenzt zumindest teilweise eine Aufnahmeöffnung 32. Die Aufnahmeöffnung 32 ist dazu eingerichtet, bei Anwendung der Atemmaske 1 zumindest Nase und Mund des Anwenders aufzunehmen. Sodann liegt der Maskenwulst 12 atemgasdicht zum Gesicht des Anwenders an und die Atemmaske 1 insgesamt umschließt zumindest Nase und Mund des Anwenders. In manchen Ausführungsformen kann die Atemmaske 1 bei Anwendung neben Mund und Nase auch das Kinn des Anwenders teilweise oder vollständig umschließen.

In einer beispielhaften Ausführungsform sind zumindest Maskenkörper 10 und Maskenflügel 14 einstückig ausgeführt. Maskenwulst 12 und Maskenkörper 10 können ebenfalls einstückig ausgeführt sein. In einer beispielhaften konkreten Ausführungsform können Maskenkörper 10, Maskenwulst 12 und Maskenflügel 14 in einem Herstellungsverfahren hergestellt werden und einstückig ausgeführt sein. Die Einstückigkeit kann auch dadurch erreicht werden, dass ein oder mehrere Teile einzeln hergestellt werden und nachträglich irreversibel miteinander verbunden werden. Eine irreversible Verbindung kann beispielsweise durch Kleben, Umspritzen, Schweißen oder anderen Verbindungsmethoden erreicht werden.

Auch eine zweistückige oder mehrstückige Ausführung ist möglich. Beispielsweise kann der Maskenwulst 12 in manchen Ausführungsformen in einem gesonderten Herstellungsschritt gefertigt werden und reversibel auf den Maskenkörper 10 aufgesteckt werden (nicht gezeigt).

Das Filterelement 40 wird in einer bevorzugten Ausführungsform reversibel mit der Atemmaske 1 verbunden. Zu diesem Zweck kann das Filterelement 40 mit der Aufnahmeeinrichtung 16 des Maskenflügels 14 verbunden werden (siehe beispielsweise Fig. 1 A, 2 A).

Die vorgenannten Bauteile der Atemmaske 1, nämlich Maskenkörper 10, Maskenwulst 12 und Maskenflügel 14, Aufnahmeeinrichtung 16 sowie Filterelement 40, sind bevorzugt aus einem Kunststoff hergestellt. Der Kunststoff kann elastisch bzw. elastomer und/oder formstabil sein. Geeignete Kunststoffe können ausgewählt sein aus der Gruppe Polycarbonate (PC), Polystyrole (PS), Polyamide (PA), Polypropylene (PP), Polyoxymethylene (POM), Polyacrylnitrile (PAN), Thermoplastische Elastomere (TPE), Acrylnitril-Styrol-Acrylester (ASA), Acrylnitril-Butadien-Styrole (ABS), Styrol-Acrylnitrile (SAN), Styrol-Methyl-Methacrylate (SMMA), Polyethylene (PE), Cycloolefin-Copolymere (COC), Polytetrafluorethylen (PTFE), Silikone. Bei der Auswahl geeigneter Materialien ist zu beachten, dass diese - mit Ausnahme des Filterelements 40 bzw. mit Ausnahme von einzelnen Bauteilen des Filterelements 40 wie beispielsweise des Filtermaterials 70- nicht für Atemgas durchlässig sind.

Die Bauteile der Atemmaske 1 können aus ein und demselben Material oder aus unterschiedlichen Materialen hergestellt werden. In einem konkreten Ausführungsbeispiel können Maskenkörper 10, Maskenwulst 12 und Maskenflügel 14 inklusive Aufnahmeeinrichtung 16 aus einem elastomeren Kunststoff hergestellt sein. Beispielsweise sind Maskenkörper 10, Maskenwulst 12 und Maskenflügel 14 inklusive Aufnahmeeinrichtung 16 aus Silikon. Die Filterelemente 40 können im Wesentlichen aus einem formstabilen Kunststoff hergestellt sein. Beispielsweise sind die Filterelemente 40 im Wesentlichen aus Polypropylen.

Durch die Wahl eines elastomeren Kunststoffes kann der Maskenkörper 10 in sich formfest sein, aber bei Zug- und/oder Druckbelastung elastisch verfombar sein. Zusätzlich zu dem Grundmaterial der einzelnen Bauteile können weitere Werkstoffe verwendet werden. Beispielsweise ist es denkbar, dass in das Grundmaterial weitere Materialien zur Verstärkung und/oder Versteifung eingebracht und/oder aufgebracht sind.

Das Grundmaterial von Maskenkörper 10, Maskenwulst 12 und Maskenflügel 14 inklusive Aufnahmeeinrichtung 16 kann beispielsweise Silikon sein. Maskenkörper 10, Maskenwulst 12, Maskenflügel 14 und Aufnahmeeinrichtung 16 können somit elastisch verformbar sein. Die Shore-Härte des verwendeten Materials liegt beispielsweise zwischen 30 und 60 Shore A, wobei eine maximale Dehnbarkeit zwischen 100% und 800% erreicht werden kann.

Eine einstückige Ausführung der Atemmaske 1, insbesondere aus Silikon, bietet den Vorteil, dass die Atemmaske 1 einfach, sicher und mehrmals gereinigt bzw. desinfiziert und/oder anderweitig aufbereitet werden kann, ohne dass die Atemmaske 1 Qualitätseinbußen erfährt. Silikon weist eine gute Chemikalien- und Temperaturbeständigkeit auf und eignet sich demnach gut für eine häufige Reinigung bzw. Desinfektion. Insbesondere ist die Atemmaske 1 ohne Filterelemente 40 autoklavierbar. Zudem ist Silikon biologisch gut verträglich.

Die Atemmaske 1 und insbesondere der Maskenkörper 10 ist bevorzugt aus einem klarsichtigen bzw. durchsichtigen Material, beispielsweise einem klarsichtigen Silikon, gefertigt. Das Material des Maskenkörpers 10 weist bevorzugt eine Lichtdurchlässigkeit von mindestens 50% auf, bevorzugt von mindestens 80%. In einem konkreten Ausführungsbeispiel beträgt die Lichtdurchlässigkeit mindestens 90%.

Die Verwendung eines klarsichtigen Materials für die Atemmaske 1 und die Anordnung des Filterelements 40 im Bereich der Maskenflügel 14 bietet den Vorteil, dass insbesondere der Mund des Anwenders bei Verwendung der Atemmaske 1 sichtbar ist. Dies kann die optische Wirkung der Atemmaske 1 positiv beeinflussen, da Gesichtsausdrücke des Anwenders von der Umgebung besser wahrgenommen werden können. Zudem bietet die Atemmaske 1 den Vorteil, dass der Mund beim Sprechen sichtbar ist, was eine Verständlichkeit der Sprache erhöhen kann.

Die Verwendung eines elastisch verformbaren Materials für die Atemmaske 1 bietet den Vorteil, dass die Atemmaske 1 insgesamt - mit Ausnahme des Filterelements 40 - weich ist. Dadurch kann sich die Atemmaske 1 um das Kinn biegen und sich so bevorzugt jeder Gesichtsform anpassen. Die Atemmaske 1 kann sich im den Nasenbereich überspannenden Abschnitt bei hohen Nasenrücken verformen und ist so für verschiedene Nasenformen geeignet. Optional kann in dem den Nasenbereich überspannenden Abschnitt ein biegbares, versteifendes Element wie beispielsweise ein Draht oder dergleichen angeordnet sein. Über dieses Element kann eine bessere Anpassung an die Nase gewährleistet werden.

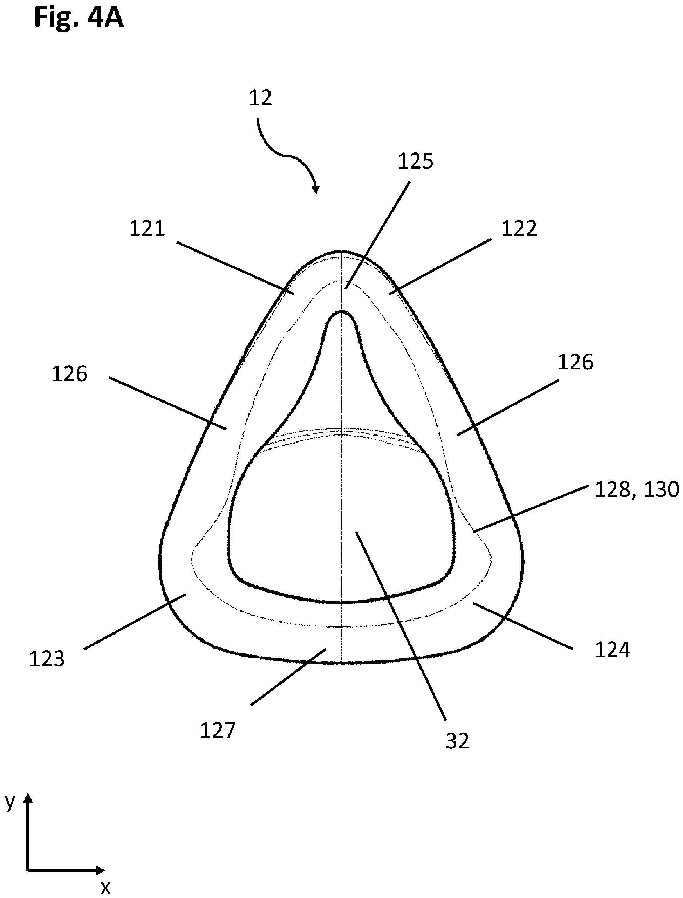

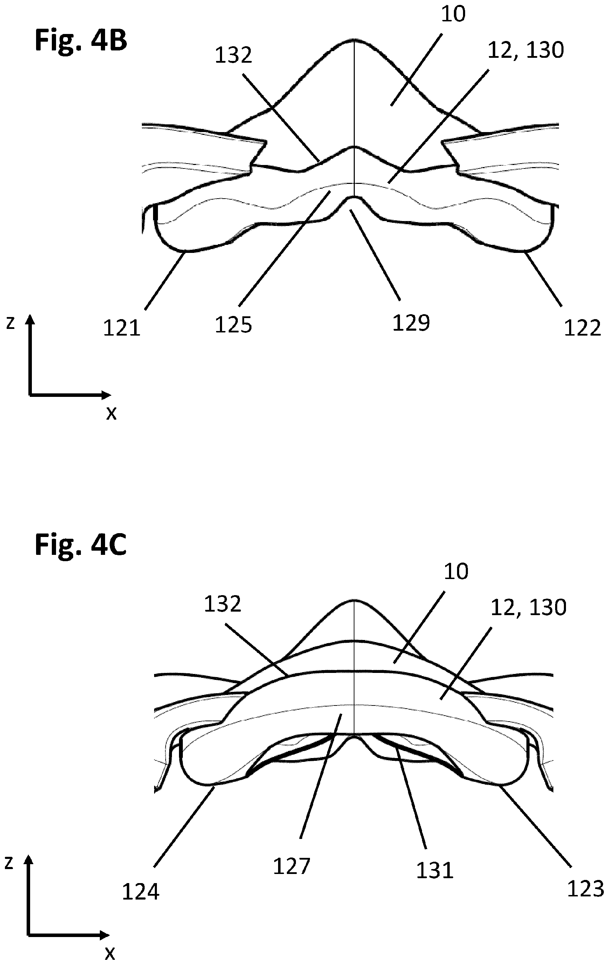

**Figur 4A****,** **4B und 4C** zeigen Darstellungen eines beispielhaften Maskenwulstes 12 der Atemmaske 1 aus unterschiedlichen Perspektiven, wobei Fig. 4A den Maskenwust 12 in Frontansicht, also die dem Anwender zugewandte Seite zeigt, Fig. 4B den Maskenwulst von oben und Fig. 4C den Maskenwulst von unten zeigt.

Der Maskenwulst 12 kann untergliedert sein in zwei Seitenbereiche 126, einen Basisbereich 127 sowie einen Nasenrückenbereich 125. Mit den Seitenbereichen 126, dem Basisbereich 127 sowie den Nasenrückenbereich 125 begrenzt der Maskenwulst 12 die Aufnahmeöffnung 32 zu den Seiten. Die Aufnahmeöffnung 32 ist dazu ausgelegt zumindest Nase und Mund des Anwenders zu umschließen. Die Aufnahmeöffnung 32 kann in manchen Ausführungsformen zusätzlich dazu ausgelegt sein, das Kinn des Anwenders zumindest teilweise aufzunehmen und/oder zu umschließen.

Der Maskenwulst 12 kann als Dichtlippe 130 ausgeführt sein und einen Auflagebereich 128 (siehe Fig. 4A) sowie eine Kante 131 (siehe Fig. 4C) aufweisen. Der Auflagebereich 128 stellt die dem Anwender zugewandte Seite dar. Der Maskenwulst 12 liegt über den Auflagebereich 128 der Dichtlippe 130 zum Gesicht des Anwenders an und schließt atemgasdicht ab. Die Dichtlippe 130 ragt beispielsweise ausgehend von den Seitenbereichen 126, dem Basisbereich 127 sowie dem Nasenrückenbereich 125 in einem Bogen in die Aufnahmeöffnung 32 hinein (siehe Fig. 4B/4C). Dabei stellt der Auflagebereich 128 gleichzeitig die vordere, d.h. die zum Anwender zugewandte Seite und die Begrenzung der Aufnahmeöffnung 32 durch den Maskenwulst 12 dar.

In dem in Fig. 4A-C beispielhaft gezeigten Maskenwulst 12 sind zusätzlich Eckpunkte 121, 122, 123 und 124 ausgebildet, welche beispielsweise gegenüber dem restlichen Maskenwulst 12 verfestigt sind oder eine erhöhte Wandstärke aufweisen. Diese Eckpunkte 121, 122, 123, 124 sind beispielsweise so angeordnet, dass sie an weniger empfindlichen Punkten im Gesicht des Anwenders aufliegen und so den Maskenwulst 12 zusätzlich abstützen. Durch die abstützende Funktion der Eckpunkte 121, 122, können beispielsweise auch andere Bereiche des Maskenwulstes 12 mit einer geringeren Wandstärke ausgeführt sein. Beispielsweise kann der Maskenwulst 12 im Nasenrückenbereich 125 eine deutlich geringere Wandstärke aufweisen. Das kann dazu führen, dass in diesem Bereich der Maskenwulst 12 nicht gegenüber dem Gesicht abgestützt wird und flexibler eingerichtet ist. Dadurch kann sich der Maskenwulst 12 in diesem Bereich einfacher der Gesichtskontur anpassen ohne einen zusätzlichen Druck im empfindlichen Bereich des Nasenrückens des Anwenders auszuüben.

Alternativ oder zusätzlich zu der geringeren Wandstärke kann im Nasenrückenbereich 125 eine Einkerbung 129 angeordnet sein (Fig. 4B), welche zusätzlich eine einfachere Anpassung an die Gesichtskonturen des Anwenders ermöglicht.

Zwischen Maskenkörper 10 und Maskenwulst 12 kann ein Übergang 132 ausgebildet sein. Maskenwulst 12 und Maskenkörper 10 können in einer beispielhaften Ausführungsform gemäß der Figuren einstückig gefertigt sein. Bei einer einstückigen Fertigung kann der Übergang 132 nicht merkbar verlaufen. Allerdings kann in manchen Ausführungsformen am Übergang 132 auch eine Änderung in Materialbeschaffenheit und/oder Materialstärke eingeführt werden.

Der Maskenwulst 12 kann in manchen Ausführungsformen auch separat bereitgestellt und zur Verwendung mit dem Maskenkörper 10 verbunden werden. Bei einer separaten Fertigung des Maskenwulstes 12, beispielsweise wenn der Maskenkörper 10 aus einem formstabilen Kunststoff vorgesehen ist und der Maskenwulst 12 aus einem elastomeren Kunststoff, kann der Maskenkörper 10 am Übergang 132 einen Maskenanschluss aufweisen, der beispielsweise so eingerichtet und ausgebildet ist, dass der Maskenwulst 12 atemgasdicht auf den Maskenkörper 10 aufgesteckt werden kann (nicht gezeigt). Die gezeigte Form des Maskenwulstes 12 ist als eine beispielhafte Ausführung eines Maskenwulstes 12 für die erfindungsgemäße Atemmaske 1 zu verstehen. Variationen der Dichtlippe 130 bzw. des Auflagebereichs 128 des Maskenwulstes 12 sind möglich. Der Maskenwulst 12 ist durch das bevorzugt elastomere bzw. weiche Material derart eingerichtet und ausgebildet, dass sich bei einer Anwendung der Atemmaske 1 eine Veränderung der durch den Auflagebereich 128 bestimmten Fläche der Aufnahmeöffnung 32 einstellen kann. Auch kann der Auflagebereich 128 bei Anwendung je nach Gesichtskontur des Anwenders variieren.

Die Materialstärke des Maskenwulstes 12 kann konstant ausgebildet sein oder zumindest bereichsweise variieren. Insgesamt hängt die Materialstärke des Maskenwulstes 12 davon ab, an welcher Stelle des Gesichts des Anwenders der jeweilige Bereich aufliegt. So ist die Materialstärke im Bereich empfindlicherer Stellen des Gesichts geringer zu wählen. In Bereichen, die an weniger empfindlicheren Stellen des Gesichts aufliegen, kann hingegen eine größere Materialstärke gewählt werden, um den Maskenwulst 12 zu stabilisieren. Beispielsweise beträgt die Materialstärke des Maskenwulstes 3 im Bereich des Nasenrückenbereiches 125 zwischen 0,1 mm und 0,8 mm, bevorzugt zwischen 0,3 mm und 0,5 mm.

Die Materialstärke im Basisbereich 127 des Maskenwulstes 12 kann vom Übergang 132 her kommend zunächst größer sein und in Richtung Kante 131 des Maskenwulstes 3 abnehmen. Im Bereich des Übergangs 132 ist die Materialstärke beispielsweise zwischen 0,5 mm und 3,0 mm, bevorzugt zwischen 0,7 mm und 2,5 mm. Zur Kante 131 hin reduziert sich die Materialstärke auf zwischen 0,1 mm und 0,8 mm. Ähnliche Verläufe der Materialstärken können beispielsweise auch in den Seitenbereichen 126 des Maskenwulstes 12 ausgebildet sein.

Der Maskenwulst 12 kann eingerichtet sein, bei Anwendung der Atemmaske 1 zumindest bereichsweise am Kinn anzuliegen. Insbesondere der Basisbereich 127 des Maskenwulstes 12 kann in manchen Ausführungsformen derart am Gesicht eines Anwenders anliegen, dass das Kinn von der Atemmaske 1 umschlossen ist. Ein derartiger Kinnumgriff kann durch eine spezielle Formgebung des Basisbereiches 127 eingerichtet sein. Beispielsweise kann der Basisberiech 127 derart konkav geformt sein, dass bei Anwendung der Atemmaske 1 das Kinn eines Anwenders in den Basisbereich 127 aufgenommen wird.

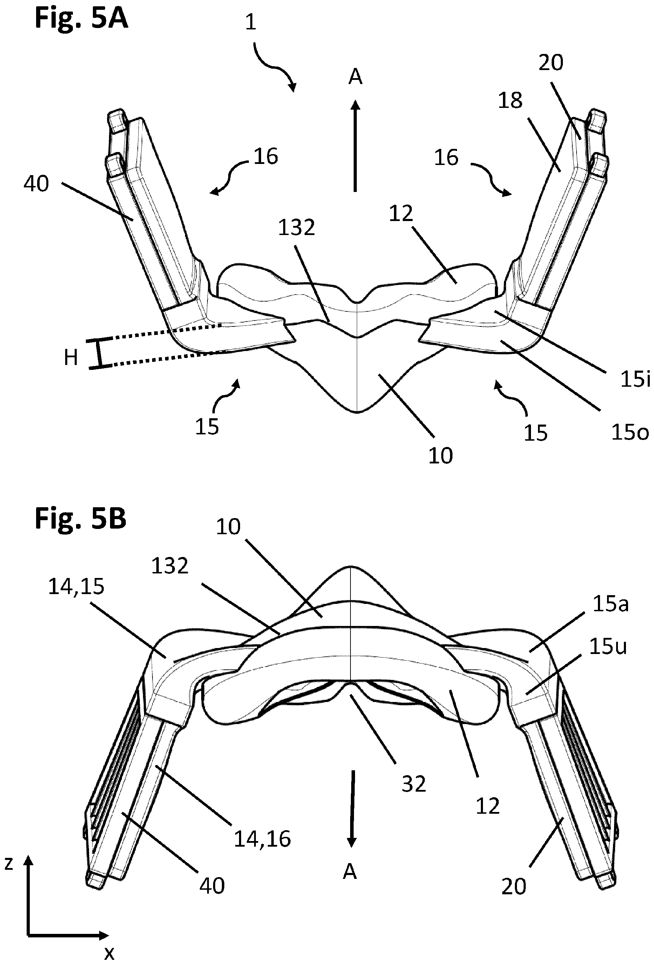

**Fig. 5A** zeigt eine Darstellung einer Atemmaske 1 mit Filterelementen 40 von oben und **Fig. 5B** eine Atemmaske 1 mit Filterelementen 40 von unten. Die Atemmaske 1 umfasst in der Regel zwei Maskenflügel 14 mit jeweils einer Aufnahmeeinrichtung 16 und mindestens einem Flügelkanal 15.

In manchen Ausführungsformen ist es auch denkbar, dass die Atemmaske 1 mehr als zwei Maskenflügel 14 aufweist, beispielsweise vier oder mehr (nicht gezeigt). Die Maskenflügel 14 sind bevorzugt ein integraler Bestandteil der Atemmaske 1. Maskenflügel 14 und Maskenkörper 10 sind bevorzugt einstückig ausgebildet. Die Maskenflügel 14 sind an den Maskenkörper 10 angeformt. Die Maskenflügel 14 sind am Maskenkörper 10 bevorzugt maximal weit entfernt von der Symmetrieebene E angeordnet (siehe Fig. 1).

Aus Fig. 5A/5B wird ersichtlich, dass die Maskenflügel 14 zwei Teilbereiche umfassen, nämlich mindestens einen Flügelkanal 15 und mindestens eine Aufnahmeeinrichtung 16.

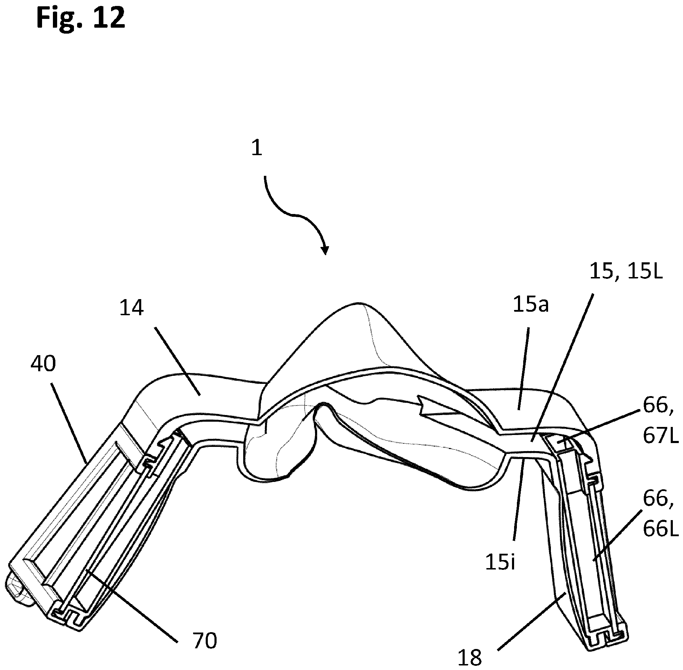

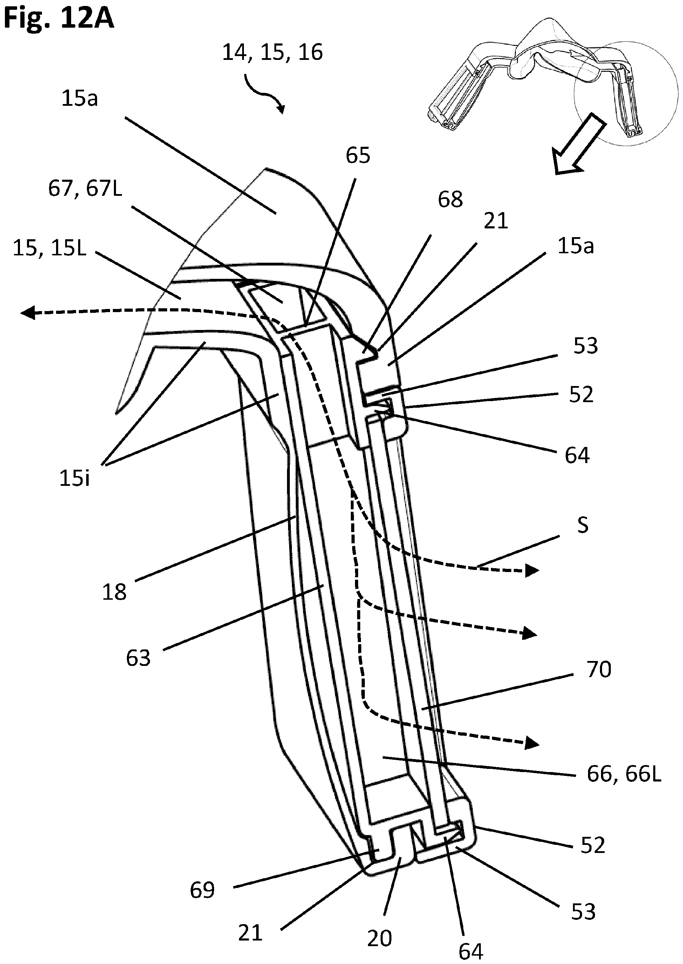

Der Flügelkanal 15 kann in Form eines Hohlkörpers ausgebildet sein und ein Lumen 15L aufweisen (siehe Fig. 12A). Der Flügelkanal 15 kann einen mehreckigen, ovalen oder runden Querschnitt aufweisen. In einer beispielhaften Ausführungsform kann der Flügelkanal 15 einen viereckigen Querschnitt aufweisen. Die Ecken können eckig oder abgerundet ausgebildet sein. Der Maskenflügel 14 im Bereich des Flügelkanals 15 kann somit in Form eines Vierkantrohrs ausgebildet sein.

Aus dem beispielhaften Ausführungsbeispiel gemäß der Figuren wird ersichtlich, dass der Flügelkanal 15 durch den bevorzugt viereckigen Querschnitt vier Seitenwände aufweisen kann: eine Außenwand 15a, eine Innenwand 15i, eine Oberwand 15o und eine Unterwand 15u.

Die Innenwand 15i (siehe Fig. 5A) weist bei Anwendung der Atemmaske 1 generell in Richtung zum Anwender. Bei Anwendung weist die Innenwand 15i in Richtung der Gesichtshaut des Anwenders. Die Außenwand 15a (siehe Fig. 5B) weist bei Anwendung der Atemmaske 1 vom Anwender weg.

Außenwand 15a und Innenwand 15i sind beabstandet voneinander. Der Abstand zwischen der Innenwand 15i und der Außenwand 15a definiert eine Höhe H des Flügelkanals 15 (siehe Fig. 5A).

Die Außenwand 15a kann zumindest teilweise parallel zu der Innenwand 15i verlaufen. In dem Fall kann die Höhe H gleichbleibend sein. Die Höhe H kann auch zu- und/oder abnehmen. In dem konkreten Ausführungsbeispiel gemäß der Figuren kann die Höhe H beispielsweise in weiten Teilen konstant verlaufen und in Richtung der Aufnahmeeinrichtung 16 zunehmen.

Oberwand 15o (siehe Fig. 5A) und Unterwand 15u (siehe Fig. 5B) sind beabstandet voneinander. Der Abstand zwischen der Oberwand 15o und der Unterwand 15u definiert eine Breite B des Flügelkanals 15 (siehe Fig. 1B). Die Breite B kann gleichbleibend sein oder zumindest abschnittsweise zu- und/oder abnehmen.

Der Flügelkanal 14 kann im Bereich, der benachbart zum Maskenkörper 10 liegt, eine erste Breite B1 aufweisen und im Bereich, der benachbart zur Aufnahmeeinrichtung 16 liegt, eine zweite Breite B2 aufweisen. In beispielhaften Ausführungsformen gemäß der Figuren kann die erste Breite B1 größer als die zweite Breite B2 sein (siehe Fig. 1B). Die Breite B kann somit vom Maskenkörper 10 in Richtung zur Aufnahmeeinrichtung 16 abnehmen. In alternativen Ausführungsformen ist es auch denkbar, dass die Breite B gleichbleibt und/oder zunimmt. Eine Abnahme der Breite B im Strömungsverlauf kann den Vorteil bieten, dass der Augenbereich frei bleibt und ein freies Sichtfeld für den Anwender entsteht.

Das Lumen des Flügelkanals 15L definiert einen hydraulischen Durchmesser des Flügelkanals 15. Das Lumen des Flügelkanals 15L kann einen hydraulischen Durchmesser von mindestens 10 mm je Flügelkanal 15 aufweisen. In einer bevorzugten Ausführungsform weist das Lumen des Flügelkanals 15L einen hydraulischen Durchmesser von mindestens 12 mm auf. In einem konkreten Ausführungsbeispiel kann das Lumen des Flügelkanals 15L einen hydraulischen Durchmesser von 15 mm oder mehr aufweisen. Der hydraulische Durchmesser ist derart ausgebildet, dass der Atemwiderstand möglichst gering ist.

Der Flügelkanal 15 kann derart eingerichtet und ausgebildet sein, dass der Flügelkanal 15 sich auch unter Zug- und/oder Druckspannung nicht derart verformt, dass sich der hydraulische Durchmesser verändert. Zu diesem Zweck kann die Wandstärke des Flügelkanals 15 stärker ausgebildet sein als beispielsweise die Wandstärke des Maskenkörpers 10.

In manchen Ausführungsformen ist es auch denkbar, dass der Flügelkanal 15 in Bereichen, die einer besonderen Zug- und/oder Druckspannung standhalten müssen, verstärkt ausgebildet ist (nicht gezeigt). Die Wandstärke des Flügelkanals 15 kann somit gleichbleibend sein oder variierend ausgebildet sein.

In manchen Ausführungsbeispielen können verstärkende Elemente den Flügelkanal 15 derart verstärken, dass das Lumen des Flügelkanals 15L sich nicht oder nicht wesentlich verformen kann (nicht gezeigt). Diese Bereiche können beispielsweise eine größere Wandstärke aufweisen. Diese Bereiche können in manchen Ausführungsformen auch zusätzliche Elemente wie beispielsweise Rippen oder ähnliches aufweisen. Diese Rippen können eingerichtet und ausgebildet sein, den Flügelkanal zu stützen und/oder die Distanzen zwischen den vier Seitenwänden 15a, 15i, 15o, 15u aufrechtzuerhalten.

Derartige Stütz- und/oder Distanzrippen können beispielsweise aus dem selben Material gefertigt sein, wie der Maskenflügel 14. In manchen Ausführungsformen können derartige Stütz- und/oder Distanzrippen aus einem anderen Material gefertigt sein und/oder aufweisen. Solche verstärkenden Elemente können beispielsweise aus einem Kunststoff, insbesondere aus einem formstabilen Kunststoff gefertigt sein. Verstärkende Elemente aus Metall sind ebenfalls denkbar. Beispielsweise können Drähte zur Verstärkung in das Grundmaterial des Flügelkanals 15 eingearbeitet sein.

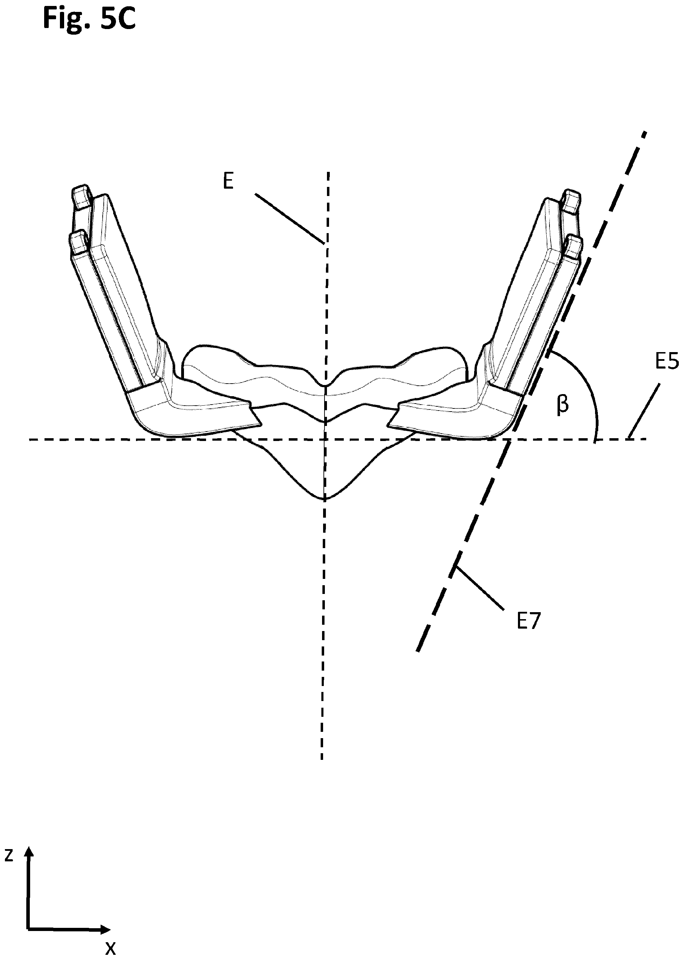

Aus Fig. 5C wird ersichtlich, dass die Maskenflügel 14 im Bereich des Flügelkanals 15 abgewinkelt und/oder gebogen ausgebildet sein können. Um dies zu veranschaulichen, sind in Fig. 5C beispielhafte Ebenen E5 und E7 angedeutet. Die Ebene E5 steht senkrecht zur Symmetrieebene E. Die Ebene E7 wird durch die Anordnung der Aufnahmeeinrichtung 16 bzw. des Filterelements 40 definiert.

Die Maskenflügel 15 können derart eingerichtet sein, dass die Ebene E7 in einem Winkel β von 30° bis 90° geneigt zu der Ebene E5 steht, wenn die Atemmaske 1 keiner Kraft von außen ausgesetzt ist. Bevorzugt kann die Ebene E7 in einem Winkel β von mindestens 45° geneigt zur Ebene E5 stehen, wenn die Atemmaske 1 keiner Kraft von außen ausgesetzt ist. In einem konkreten Ausführungsbeispiel kann die Ebene E7 in einem Winkel β von 70° geneigt zur Ebene E5 stehen, wenn die Atemmaske 1 keiner Kraft von außen ausgesetzt ist.

Die Maskenflügel 14 umfassen neben dem Flügelkanal 15 die mindestens eine Aufnahmeeinrichtung 16. Die Aufnahmeeinrichtung 16 ist bevorzugt ein integraler Bestandteil der Maskenflügel 14. Aufnahmeeinrichtung 16 und Maskenflügel 14 sind bevorzugt einstückig ausgebildet. Eine einstückige Ausführung hat den Vorteil einer einfachen Fertigung. Die Aufnahmeeinrichtung 16 ist hierbei an den Flügelkanal 15 angeformt. Somit ist der Flügelkanal 15 zwischen dem Maskenkörper 10 und der Aufnahmeeinrichtung 16 angeordnet. Die Aufnahmeeinrichtung 16 kann bevorzugt maximal weit entfernt von der Symmetrieebene E der Atemmaske 1 angeordnet sein.

In alternativen Ausführungsformen ist es auch denkbar, dass die Aufnahmeeinrichtung 16 ein lösbarer Bestandteil der Atemmaske 1 ist und beispielsweise lösbar mit dem Flügelkanal 15 verbunden werden kann (nicht gezeigt). Eine solche Ausgestaltung könnte eine effizientere Reinigung ermöglichen, da auch schwer zugängliche Bereiche erreicht werden können. Zudem könnte eine mehrteilige Ausbildung der Atemmaske 1 das Verbinden des Filterelements 40 mit der Atemmaske 1 erleichtern. Beispielsweise ist es denkbar, dass zunächst Aufnahmeeinrichtung 16 und Filterelement 40 miteinander verbunden werden und erst anschließend gemeinsam an der Atemmaske 1 bzw. am Flügelkanal 15 angebracht werden. Das kann die Handhabung der Atemmaske 1 erleichtern.

Die Aufnahmeeinrichtung 16 des Maskenflügels 14 ist eingerichtet und ausgebildet, das Filterelement 40 aufzunehmen. Die Verbindung von Filterelement 40 und Aufnahmeeinrichtung 16 kann beispielsweise durch Einpressen des formstabilen Filterelements 40 in die elastisch verformbare Aufnahmeeinrichtung 16 erfolgen. Aufnahmeeinrichtung 16 und Filterelement 40 sind zu diesem Zwecke zumindest teilweise formkomplementär ausgebildet.

Die zweidimensionale Grundform von Aufnahmeeinrichtung 16 und Filterelement 40 kann rund oder mehreckig sein. In einem beispielhaften Ausführungsbeispiel kann die Grundform von Aufnahmeeinrichtung 16 und Filterelement 40 viereckig sein. Beispielsweise sind die Grundformen von Aufnahmeeinrichtung 16 und Filterelement 40 rautenförmig (nicht gezeigt) oder trapezförmig (siehe beispielsweise Fig. 2A). Eine trapezförmige Grundform bietet den Vorteil, dass das Filterelement 40 beidseitig eingesetzt werden kann und keine Unterscheidung in "linkes" Filterelement 40 und "rechtes" Filterelement 40 vorgenommen werden muss.

Aus Fig. 5A wird weiterhin ersichtlich, dass die Aufnahmeeinrichtung 16 mindestens eine Aufnahme-Seitenwand 20 umfasst. Die Aufnahme-Seitenwand 20 ist bevorzugt elastisch verformbar. Die Aufnahme-Seitenwand 20 umläuft die beispielsweise trapezförmige Aufnahmeeinrichtung 16. In einer beispielhaften Ausführungsform ist die Aufnahme-Seitenwand 20 dreiseitig umlaufend ausgeführt, wobei an der Seite, die zum Flügelkanal 15 weist, keine Aufnahme-Seitenwand 20 ausgebildet ist.

Die Aufnahmeeinrichtung 16 kann zusätzlich zu der Aufnahme-Seitenwand 20 eine Aufnahme-Rückwand 18 umfassen. Bei einer Anlage der Atemmaske 1 am Kopf des Anwenders kann die Aufnahme-Rückwand 18 zumindest teilweise dem Gesicht zugewandt und/oder am Gesicht anliegend sein.

Die Aufnahme-Rückwand 18 kann benachbart zur Innenwand 15i des Flügelkanals 15 sein. Die Aufnahme-Rückwand 18 kann mit der Innenwand 15i des Flügelkanals 15 verbunden sein. In dem konkreten Ausführungsbeispiel gemäß der Figuren sind Innenwand 15i und Aufnahme-Rückwand 18 einstückig ausgebildet und weisen in Richtung des Anwenders. Die Aufnahme-Rückwand 18 kann die Aufnahmeeinrichtung 16 in sich stabilisieren, was die Aufnahme der Filterelements 40 in die Aufnahmeeinrichtung 16 erleichtert.

In manchen Ausführungsformen kann die Aufnahmeeinrichtung 16 auch ohne Aufnahme-Rückwand 18 ausgebildet sein. Sodann besteht die Aufnahmeeinrichtung 16 lediglich aus der Aufnahme-Seitenwand 20. Bei einer Anlage der Atemmaske 1 am Kopf des Anwenders kann sodann die Filtereinrichtung 40 zumindest teilweise dem Gesicht zugewandt und/oder am Gesicht anliegend sein. Eine Ausführungsform ohne Aufnahme-Rückwand 18 bietet den Vorteil einer Materialersparnis, was wiederrum das Gewicht der Atemmaske reduziert und Herstellungskosten senken kann.

Die Aufnahmeeinrichtung 16 ist derart eingerichtet, dass sie das Filterelement 40 aufnehmen kann. Wenn das Filterelement 40 mit der formkomplementären Aufnahmeeinrichtung 16 verbunden ist, befindet sich zumindest ein Abschnitt des Filterelements 40 innerhalb zumindest eines Bereiches des Lumens des Flügelkanals 15L. In einer beispielhaften Ausführungsform ist eine Seite des viereckig ausgebildeten Filterelements 40 zumindest abschnittsweise im Lumen des Flügelkanals 15L angeordnet. Die anderen drei Seiten des Filterelements 40 sind innerhalb der Aufnahme-Seitenwand 20 angeordnet. Somit kann das Filterelement 40 derart in die elastisch verformbare Aufnahmeeinrichtung 16 eingebracht werden, dass das Filterelement 40 innerhalb der dreiseitig umlaufenden Aufnahme-Seitenwände 20 eingepresst ist.

Zu diesem Zweck kann die Aufnahmeeinrichtung 16 so ausgeführt sein, dass diese beispielsweise maximal so groß ist wie das Filterelement 40. Beispielsweise ist die Aufnahmeeinrichtung 16 geringfügig kleiner als das Filterelement 40 ausgebildet. Das Filterelement 40 kann dann zur Herstellung eines Anwendungszustandes in die elastisch ausgebildete Aufnahmeeinrichtung 16 eingedrückt werden. Dabei verformt sich die Aufnahme-Seitenwand 20 zunächst leicht und kann nach Aufnahme des Filterelements 40 in seine ursprüngliche Form zurückkehren. Somit wird das Filterelement 40 unter leichter Spannung in der Aufnahmeeinrichtung 40 gehalten.

Die dem Flügelkanal 15 gegenüberliegende Seite der Aufnahme-Seitenwand 20 kann zudem eine Arretierungsaussparung 21 aufweisen, um das Filterelement 40 in der Aufnahmeeinrichtung 16 zu arretieren (siehe Fig. 12A).

Bevorzugt ist das Eindrücken des Filterelements 40 in die Aufnahmeeinrichtung 16 reversibel. Das Filterelement 40 kann somit in der Regel aus der Aufnahmeeinrichtung 16 wieder herausgenommen werden, beispielsweise zur Reinigung der Atemmaske 1 oder zum Wechseln des Filtermaterials 70. In manchen Ausführungsformen ist es auch denkbar, dass das Filtermaterial 70 auch gewechselt werden kann, wenn das Filterelement 40 mit der Atemmaske 1 verbunden ist.

Wenn das Filterelement 40 mit der Atemmaske 1 verbunden ist definieren Maskenkörper10 und Maskenwulst 12 und Maskenflügel 14 mit den Flügelkanälen 15 und Filterelement 40 einen Atemgasraum 2, der bei Anwendung der Atemmaske 1 im Wesentlichen atemgasdicht ist. Eine Atemgasströmung vom Mund und Nase des Anwenders weg sowie zum Mund und Nase des Anwenders hin ist bei Anwendung der Atemmaske 1 somit ausschließlich durch das Filterelement 40 und insbesondere durch das Filtermaterial 70 möglich.

Der Atemgasraum 2 weist ein Volumen auf, das derart gering ist, dass eine CO2-Auswaschung aus der Atemluft des Anwenders erfolgt. Der Atemgasraum 2 weist beispielsweise ein Volumen von maximal 500 ml auf. Das Volumen des Atemgasraums 2 weist beispielsweise ein Volumen von 50 ml bis 500 ml auf. Bevorzugt beträgt das Volumen des Atemgasraums 2 bei Anwendung der Atemmaske 1 maximal 120 ml.

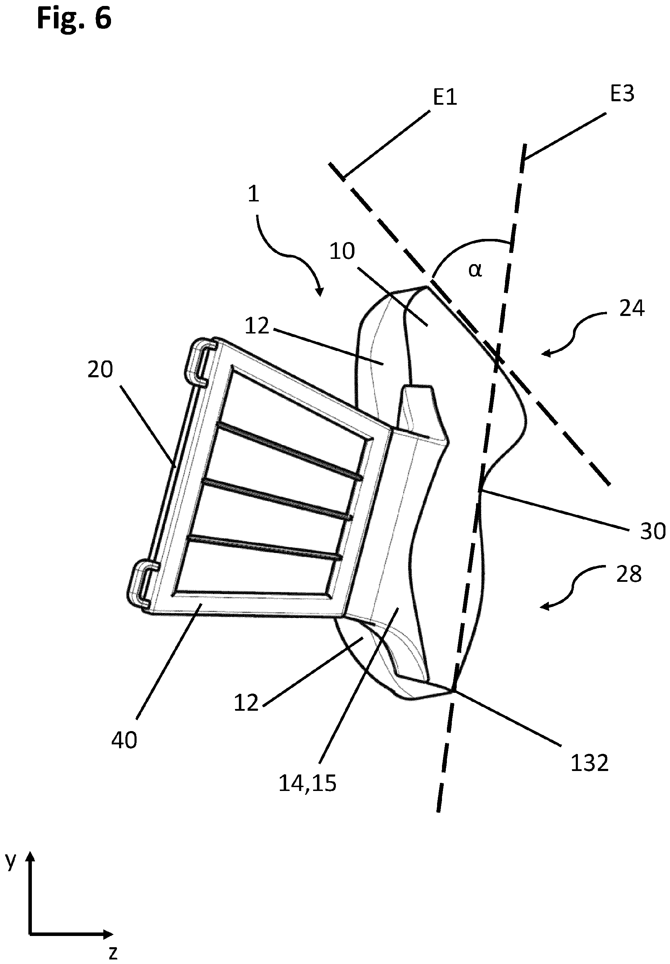

**Fig. 6** zeigt eine Darstellung einer Atemmaske 1 mit Filterelementen 40 von der Seite. Der Maskenkörper 10 ist in der Regel aus Kunststoff gefertigt. Der Maskenkörper 10 kann aus einem formstabilen und/oder elastomeren Kunststoff gefertigt sein. In einem bevorzugten Ausführungsbeispiel ist der Maskenkörper 10 aus Silikon gefertigt. Silikon ist ein elastomerer Kunststoff, der in sich formfest, aber unter Zug- und/oder Druckbelastung elastisch verformbar sein kann. Zusätzlich zum Silikon als Grundmaterial des Maskenkörpers 10 können weitere Werkstoffe verwendet werden. Beispielsweise ist es denkbar, dass in das Silikon Materialien zur Verstärkung und/oder Versteifung und/oder Abpolsterung eingebracht und/oder aufgebracht werden.

Aus Fig. 6 wird ersichtlich, dass der Maskenkörper 10 vorgeformt ist. Der Maskenkörper 10 kann im Wesentlichen einen Nasenbereich 24 und einen Mundbereich 28 aufweisen. Der Nasenbereich 24 bedeckt in einem Anwendungszustand am Kopf 90 eines Anwenders im Wesentlichen den Nasenrücken und die Nase des Anwenders. Der Mundbereich 28 überspannt in einem Anwendungszustand am Kopf 90 eines Anwenders im Wesentlichen zumindest den Mund des Anwenders.

Zwischen Nasenbereich 24 und Mundbereich 28 ist ein Anschlussbereich 30, der als Sollknickstelle ausgebildet und eingerichtet sein kann. Der Anschlussbereich 30 kann zu diesem Zwecke eine zum Nasenbereich 24 und/oder zum Mundbereich 28 variierende Materialstärke und/oder Materialbeschaffenheit aufweisen. Beispielsweise können Nasenbereich 24 und Mundbereich 28 eine höhere Materialstärke aufweisen als der Anschlussbereich 30.

In manchen Ausführungsformen können alternativ oder zusätzlich Nasenbereich 24 und Mundbereich 28 eine im Wesentlichen identische Materialeigenschaft aufweisen und der Anschlussbereich 30 demgegenüber differente Materialeigenschaften. Beispielsweise kann die die Shore-Härte und somit die maximale Dehnbarkeit in den unterschiedlichen Bereichen 24,28,30 unterschiedlich ausgebildet sein.

In manchen Ausführungsformen können im Mundbereich 28 und/oder im Nasenbereich 24 Verstärkungselemente angeordnet sein (nicht gezeigt). Derartige Verstärkungselemente können eingerichtet und ausgebildet sein, Kräfte abzufangen, die durch die Kopfanbindung über die Kopfbefestigungsbänder auf die Atemmaske 1 einwirken. Die Verstärkungselemente können in Form von Materialverdickungen ausgebildet sein. Die Verstärkungselemente können auch zusätzlich eingebrachte Elemente sein. Beispielsweise kann verstärkendes Material aus einem Hartkunststoff und/oder einem Metall und/oder einem Thermoplast und/oder einem Gewebe sein. Die Verstärkungselemente können in Form von Drähten, Bügeln, Fasern, Stäben, Streifen oder ähnlichem ausgebildet sein. In vorteilhaften Ausführungsformen können die Verstärkungselemente als verformbare Elemente ausgebildet sein, so dass die Atemmaske 1 individuell an die Kopfform des jeweiligen Anwenders angepasst werden kann

In Fig. 6 ist dargestellt, wie Nasenbereich 24 und Mundbereich 28 des Maskenkörpers 10 zueinander angeordnet sind. Zu diesem Zwecke sind beispielhafte Ebenen E1 und E3 angedeutet. Hierbei definiert der Nasenbereich 24 eine Ebene E1. Der Mundbereich 28 definiert eine Ebene E3, wobei diese durch den in der Symmetrieebene E liegenden Anschlussbereich 30 und den in der Symmetrieebene E liegenden Übergang 132 an der Basis der Atemmaske schneidet.

Der Maskenkörper 10 ist derart eingerichtet, dass die Ebene E1 in einem Winkel α von 30° bis 70° geneigt zu der Ebene E3 steht, wenn die Atemmaske 1 keiner Kraft von außen ausgesetzt ist. Bevorzugt kann die Ebene E1 in einem Winkel α von mindestens 40° geneigt zur Ebene E3 stehen, wenn die Atemmaske 1 keiner Kraft von außen ausgesetzt ist. In einem konkreten Ausführungsbeispiel kann die Ebene E1 in einem Winkel α von 48° geneigt zur Ebene E3 stehen, wenn die Atemmaske 1 keiner Kraft von außen ausgesetzt ist.

Dadurch, dass der Anschlussbereich 30 derart als Sollknickstelle ausgebildet und eingerichtet werden kann ergibt sich der Vorteil einer besseren Anpassbarkeit der Atemmaske 1 an das Gesicht eines Anwenders. Bei Anwendern mit einem flachen Nasenrücken kann die Atemmaske 1 in ihrem ursprünglich vorgeformten Zustand verbleiben. Bei Anwendern mit einem ausgeprägten bzw. hohem Nasenrücken kann sich die Atemmaske der Kontur des Gesichtes anpassen.

Bei einer Anwendung der Atemmaske 1 am Kopf kann ein hoher bzw. ausgeprägter Nasenrücken des Anwenders den Nasenbereich 24 derart verdrängen, dass sich der Winkel α zwischen Ebene E1 und Ebene E3 ändern kann. Der Winkel α zwischen Ebene E1 und Ebene E3 kann sich unter Krafteinwirkung verändern.

Der Maskenkörper 10 ist derart eingerichtet, dass der Winkel α bei einer Anwendung der Atemmaske 1 am Kopf eines Anwenders um mindestens 5° reduziert sein kann. Der Winkel α kann bei einer Anwendung der Atemmaske 1 am Kopf eines Anwenders um bis zu 25° reduziert sein.

Der Nasenbereich 24 kann in manchen Ausführungsformen versteifende und/oder abpolsternde Bereiche aufweisen. Optional kann der Nasenbereich 24 versteifende Elemente aufweisen, die zugleich formbare sind und beispielsweise quer zur Symmetrieachse E angeordnet sein können. Beispielsweise können verformbare Bügel aus Draht oder einem vergleichbaren Material eingebracht sein, so dass durch Verformung der Bügel eine optimale Anpassung des Maskenkörpers 10 an den individuellen Nasenrücken erfolgen kann.

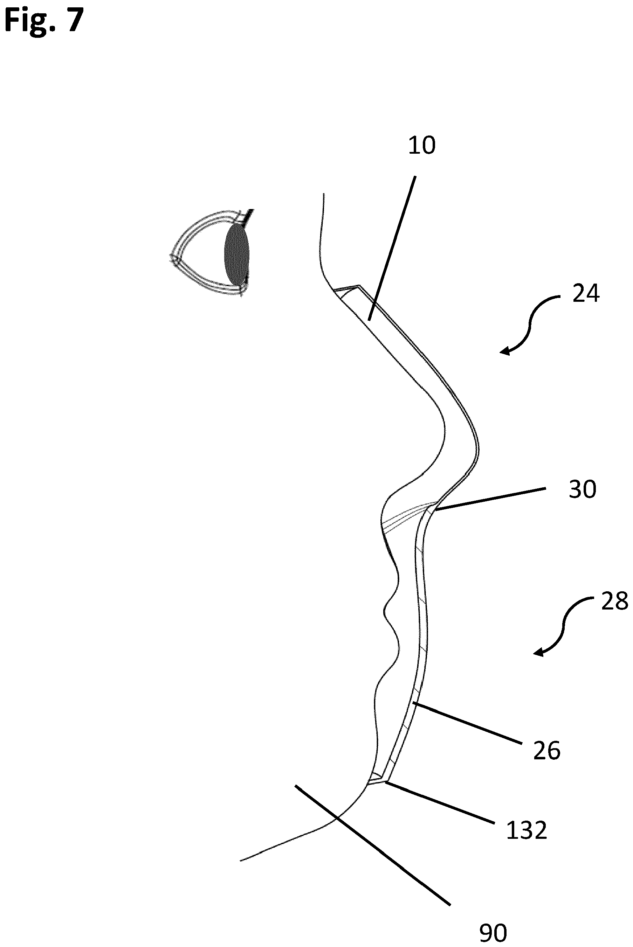

**Fig. 7** zeigt eine vereinfachte Ansicht einer Atemmaske 1 im Querschnitt bei Anlage am Kopf 90 eines Anwenders. Die Wandstärke des Maskenkörpers 10 kann gleichbleibend oder variierend ausgebildet sein. In dem in Fig. 7 gezeigten Ausführungsbeispiel wird aus dem Querschnitt deutlich, dass die Wandstärke des Maskenkörpers 10 in unterschiedlichen Bereichen 24,28 unterschiedlich stark ausgebildet sein kann.

So kann beispielsweise der Nasenbereich 24 eine gegenüber dem restlichen Maskenkörper 10 geringere Wandstärke aufweisen. Der Bereich der Nase ist in der Regel ein empfindlicher Bereich im Gesicht, so dass sich eine geringere Wandstärke hier positiv auf den Tragekomfort auswirken kann. Der Mundbereich 28 hingegen kann beispielsweise gegenüber dem Nasenbereich 24 eine erhöhte Wandstärke aufweisen. Durch die erhöhte Wandstärke im Mundbereich 28 kann die Atemmaske 1 in sich stabilisiert werden.

Die Wandstärke kann im Nasenbereich 24 in einem Bereich zwischen 0,1 und 3 mm liegen. In bevorzugten Ausführungsbeispielen kann die Wandstärke im Nasenbereich 24 in einem Bereich zwischen 0,3 und 1,5 mm liegen. In einem konkreten Ausführungsbeispiel beträgt die Wandstärke im Nasenbereich 24 0,7 mm.

Die Wandstärke kann im Mundbereich 28 in einem Bereich zwischen 0,1 und 5 mm liegen. In bevorzugten Ausführungsbeispielen kann die Wandstärke im Mundbereich 28 in einem Bereich zwischen 1 und 3 mm liegen. In einem konkreten Ausführungsbeispiel beträgt die Wandstärke im Mundbereich 28 1,5 mm.

Nasenbereich 24 und Mundbereich 28 weisen in dem konkreten Ausführungsbeispiel gemäß der Figuren jeweils eine im Wesentlichen konstante Wandstärke auf. Variierende Wandstärken innerhalb des Nasenbereichs 24 und/oder innerhalb des Mundbereichs 28 sind in manchen Ausführungsbeispielen auch denkbar.

Zwischen dem Nasenbereich 24 mit einer bevorzugt geringen Wandstärke und dem Mundbereich 28 mit einer im Gegensatz zum Nasenbereich 24 bevorzugt erhöhten Wandstärke ist der Anschlussbereich 30 angeordnet. Die Wandstärke kann im Anschlussbereich 30 in einem Bereich zwischen 0,1 und 5 mm liegen. Im Anschlussbereich 30 können der Nasenbereich 24 und der Mundbereich 28 direkt aufeinandertreffen, so dass die Wanddicke zwischen der Wanddicke des Nasenbereichs 24 und der Wanddicke des Mundbereichs 26 springt.

Dadurch, dass der Maskenwulst 12 mit seinem als Dichtlippe 130 ausgeführten Auflagebereich 128 an der Gesichtshaut atemgasdicht anliegt, sind Maskenkörper 10 und Gesicht des Anwenders räumlich voneinander getrennt. Je geringer der Abstand zwischen Maskenkörper 10 und dem Gesicht des Anwenders ist, desto geringer ist das Totraumvolumen. Durch die Formgebung der Atemmaske 1 kann das Totraumvolumen sehr gering ausgebildet sein. Das Totraumvolumen ist das Volumen, das sich in einem Anwendungszustand zwischen der Atemmaske 1 und der Oberfläche des Gesichts ausbildet. Aus Fig. 7 wird ersichtlich, dass die Atemmaske 1 in weiten Teilen, insbesondere im Nasenbereich 24 und im Mundbereich 28 jeweils eine äquidistante Fläche zum Gesicht des Anwenders ausbildet. Somit bietet die erfindungsgemäße Atemmaske 1 den Vorteil, dass durch das geringe Totraumvolumen die Gefahr der CO₂-Rückatmung für den Anwender sehr gering ist. Das Totraumvolumen der Atemmaske 1 ist in bevorzugten Ausführungsbeispielen geringer als 200 ml. Beispielsweise ist das Totraumvolumen der Atemmaske 1 geringer als 120 ml.

Der Abstand zwischen dem Maskenkörper 10 und dem Gesicht des Anwenders ist durchschnittlich geringer als 20 mm. In manchen Ausführungsformen ist der Abstand zwischen dem Maskenkörper 10 und dem Gesicht des Anwenders durchschnittlich geringer als 15 mm. Beispielsweise ist der Abstand zwischen dem Maskenkörper 10 und dem Gesicht des Anwenders durchschnittlich geringer als 10 mm.

Die erfindungsgemäße Ausbildung des Maskenkörpers 10 kann auch auf andere Atemmasken übertragen werden. Beispielsweise ist es denkbar, dass Atemmasken zur Beatmung und/oder zur Atemunterstützung mit dem erfindungsgemäßen Maskenkörper 10 ausgestattet sind, um eine optimale Anpassung an die Gesichtskontur des Anwenders zu erhalten.

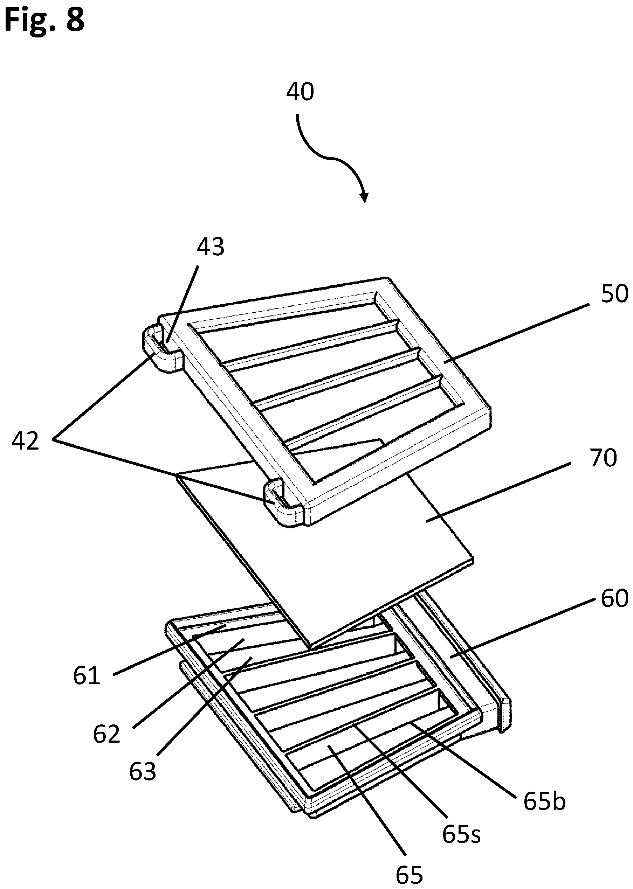

**Fig. 8** zeigt eine Explosionsansicht eines Filterelements 40. Aus Fig. 8 wird ersichtlich, dass das Filterelement drei hauptsächliche Bauteile umfassen kann, nämlich ein Vorderteil 50, ein Hinterteil 60 und ein Filtermaterial 70. Die Grundform des Filterelements 40 - und somit auch seiner Bauteile 50, 60, 70 - kann im Wesentlichen bevorzugt viereckig, beispielsweise trapezförmig, ausgebildet sein. Eine rautenförmige, quadratische, runde, halbkreisförmige oder eine Freiform-Ausbildung ist auch denkbar (nicht gezeigt). Das Filterelement 40 ist formkomplementär zur Aufnahmeeinrichtung 16 ausgebildet, so dass das Filterelement 40 zur Herstellung eines Anwendungszustandes der Atemmaske 1 in die Aufnahmeeinrichtung 16 eingepresst werden kann.

Das partikelfiltrierende Filtermaterial 70 kann in einer einfachen Ausführungsform flach ausgebildet sein. Das Filtermaterial 70 kann einlagig oder mehrlagig verwendet werden. Das Filtermaterial 70 kann in manchen Ausführungsformen auch gewellt oder Ziehharmonika-artig geknickt ausgebildet sein. Dadurch kann die Filteroberfläche erhöht werden, ohne die Fläche des Filterelements 40 zu vergrößern. In einer weiteren Ausführungsform kann das Filtermaterial 70 beispielsweise aus einem gasdurchlässigen Behälter oder Sack bestehen, welcher mit einem Filterstoff, z.B. Aktivkohle, gefüllt ist.

Das Filtermaterial 70 kann eine Höhe, eine Breite und eine Dicke aufweisen. Das Filtermaterial 70 kann im Wesentlichen flach ausgebildet sein. Die Dicke des Filtermaterials 70 kann um ein vielfaches geringer ausgebildet sein als seine Höhe und Breite. Die Höhe und Breite des Filtermaterials 70 bilden eine Fläche des Filtermaterials 70 aus, durch die der Gasaustausch bei Anwendung der Atemmaske 1 zumindest bereichsweise erfolgen kann.

Das Filtermaterial 70 kann eine Fläche von mindestens 5 cm² aufweisen. In manchen Ausführungsformen kann die Fläche des Filtermaterials 70 mindestens 30 cm² betragen. In einer bevorzugten Ausführungsform kann die Fläche des Filtermaterials 70 mindestens 50 cm² betragen.

Das Filtermaterial 70 kann zwischen dem Vorderteil 50 und dem Hinterteil 60 angeordnet sein. Das Filterelement 40 ist derart eingerichtet, dass das Filtermaterial 70 einfach und schnell zu entnehmen ist. Sodann kann das Filtermaterial 70 ersetzt und/oder gereinigt werden.

Vorderteil 50 und Hinterteil 60 können zu diesem Zwecke zweiteilig und formkomplementär ausgebildet sein. Vorderteil 50 und Hinterteil 60 können derart ausgebildet sein, dass sie reversibel miteinander verbunden werden können. Die Verbindung von Vorderteil 50 und Hinterteil 60 kann beispielsweise über ein Einpressen erfolgen. Beispielsweise können Vorderteil 50 und Hinterteil 60 derart ausgebildet sein, dass das Vorderteil 50 zumindest bereichsweise in das Hinterteil 60 reversibel eingepresst werden kann und vice versa. In manchen Ausführungsformen kann das Vorderteil 50 auch ohne Einpressen mit dem Hinterteil 60 verbunden werden und beispielsweise über zusätzliche Halteelemente, z.B. kleine Haken, an dem Hinterteil 60 befestigt werden.

In einer alternativen, nicht gezeigten Ausführungsform ist es auch möglich, dass Vorderteil 50 und Hinterteil 60 zumindest bereichsweise dauerhaft miteinander verbunden sind. Es ist zum Beispiel denkbar, dass Vorderteil 50 und Hinterteil 60 über eine Knickverbindung, beispielsweise ein Scharnier, an einer Seite miteinander verbunden sind. So könnte das Filterelement 40 insgesamt mit seinem Vorderteil 50 und seinem Hinterteil 70 aufklappbar ausgebildet sein, um das Filtermaterial 70 einzulegen bzw. zu wechseln.

Das Filterelement 40 befindet sich in einem Anwendungszustand, wenn Vorderteil 50 und Hinterteil 70 miteinander verbunden sind und das Filtermaterial 70 umschließen.

In einem konkreten bevorzugten Ausführungsbeispiel besteht die Atemmaske 1 aus einem Silikonteil, in das bevorzugt zwei Filterelemente 40 aus einem im wesentlichen formstabilen Kunststoff eingesetzt werden, die jeweils ein Filtermaterial 70 enthalten. Die Kopfanbindung der Atemmaske 1 kann über einfache Bänder erfolgen. Zu diesem Zweck kann die Atemmaske 1 mindestens eine Bänderungshalterung 42 umfassen.

Die Bänderungshalterung 42 kann am Filterelement 40 (siehe Figuren 8 ff.) oder an den Maskenflügeln 14 (nicht gezeigt) angeordnet sein. Eine Anordnung der Bänderungshalterung 42 am formstabilen Filterelement 40 bietet den Vorteil, dass die Bänderung stabil gehalten wird und die Atemmaske 1 auch unter Zug- und/oder Druckkraft durch die Bänderung keine wesentliche Formveränderung erfährt.

Die Bänderungshalterung 42 kann beispielsweise in Form eines Vorsprunges ausgebildet sein, welcher mit zumindest einer Durchführungsöffnung 43 versehen ist. Durch die Durchführungsöffnung 43 kann ein nicht gezeigtes Kopfbefestigungsband geführt werden und zum Beispiel mit der Bänderungshalterung 42 verknotet oder verklemmt werden. Ein Einklemmen kann beispielsweise dadurch erzielt werden, dass die Durchführungsöffnung 43 konisch oder trichterförmig ausgeführt ist und in Richtung vom Anwender weg verjüngend, aber nicht verschließend, angeordnet ist. Durch die Trichterform kann ein einfaches Einfädeln ermöglicht werden, jedoch die entgegengesetzte Zugrichtung gesperrt oder zumindest stark erschwert werden. Alternativ kann das Kopfbefestigungsband auch mit einem Klettverschluss ausgestattet, sodass das Kopfbefestigungsband an sich selbst befestigt wird und eine Längeneinstellung möglich wird.

Mit dem Kopfbefestigungsband kann ein fester Sitz der Atemmaske 1 auf dem Gesicht des Anwenders gewährleistet werden. In einer beispielhaften Ausführungsform ist das Kopfbefestigungsband ein elastisches Band, welches in einem gedehnten Zustand über bzw. hinter dem Kopf des Anwenders verläuft und dann in einem weniger gedehnten Zustand die Atemmaske 1 leicht an das Gesicht des Anwenders andrückt, um diese gegenüber dem Gesicht atemgasdicht abzuschließen.

Das Filterelement 40 kann in einer bevorzugten Ausführungsform die mindestens eine Bänderungshalterung 42 umfassen. In der beispielhaften Ausführungsform gemäß der Figuren ist die Bänderungshalterung am Vorderteil 50 des Filterelements 40 angeordnet (siehe Fig. 8 ff.). Eine Anordnung am Hinterteil 60 ist in alternativen Ausführungsformen auch denkbar.

Die Atemmaske 1 enthält mindestens zwei, bevorzugt mindestens vier oder mehr Bänderungshalterungen 42. In einem konkreten Ausführungsbeispiel sind in jeder der beiden spiegelsymmetrischen Hälften der Atemmaske 1 jeweils zwei Bänderungshalterungen 42 angeordnet. Bevorzugt sind jeweils zwei Bänderungshalterungen 42 an jeweils einem Filterelement 40 angeordnet. In einem konkreten Ausführungsbeispiel sind die zwei Bänderungshalterungen 42 voneinander beabstandet am Vorderteil 50 des Filterelements 40 angeordnet.

Um zu verhindern, dass sich die Atemmaske 1 unter den Druck- und/oder Zugkräften unerwünscht verformt ist es in manchen Ausführungsformen auch denkbar, dass in der Atemmaske 1 Verstärkungselemente eingebracht sind, die eine Verformung unter Krafteinwirkung verhindern.

Beispielsweise können in einem Bereich, der in einem Anwendungszustand unter der Nase anliegt, Verstärkungsbügel angeordnet sein. Diese sind somit zwischen den beiden oberen Bänderungshalterungen 42 angeordnet und können die Kräfte durch die Querverbindung abfangen. Alternativ oder zusätzlich können auch in einem Bereich, der in einem Anwendungszustand am oder unterm Kinn anliegt, Verstärkungsbügel angeordnet sein, da die Kraftdurchleitung an der unteren Maskenkante verlaufen kann. Ein Kinnumgriff an der Basis der Atemmaske 1 ist auch denkbar (nicht gezeigt).

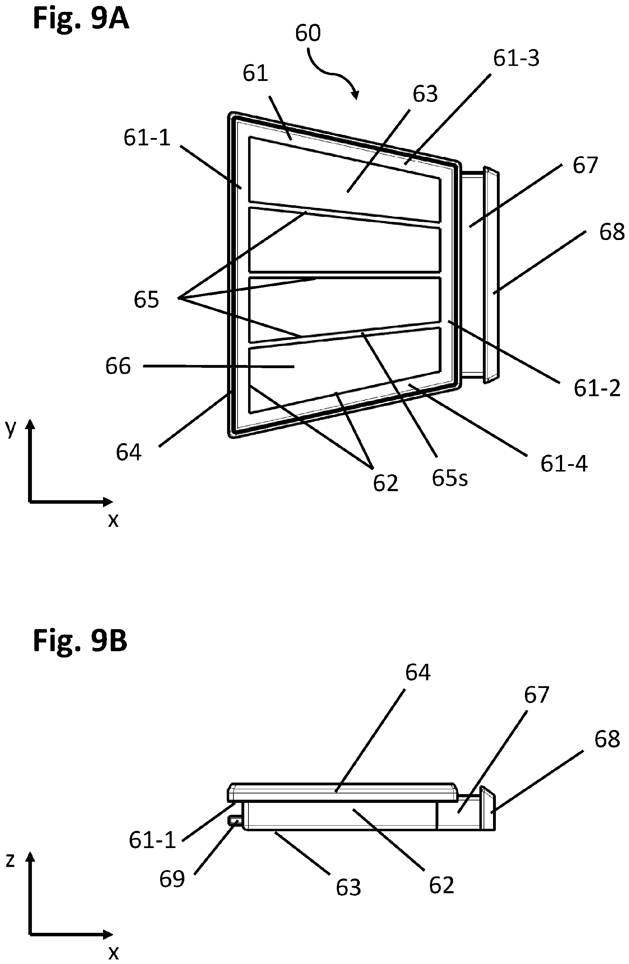

**Fig. 9 A-E** zeigen ein Hinterteil 60 eines Filterelements 40 aus unterschiedlichen Perspektiven. Die dargestellten Koordinatensysteme dienen der Verdeutlichung der Ausrichtung der Ansichten.

Das Hinterteil 60 umfasst mindestens einen Luftraum, der in Form von zumindest einem Kanal 66 ausgebildet sein kann. Der Kanal 66 ist durch eine Rückwand 63 des Hinterteils 60 und - zumindest bereichsweise - durch mindestens eine Seitenwand 62 begrenzt. Die Seitenwand 62 ist in der Regel senkrecht zur Rückwand 63 angeordnet. Rückwand 63 und Seitenwand 62 stellen einen Grundkörper des Hinterteils 60 dar und definieren den Kanal 66 zumindest bereichsweise. Rückwand 63 und Seitenwand 62 können in einfachen Ausführungsformen eine glatte Oberfläche aufweisen. In manchen Ausführungsformen sind strukturierte Oberflächen denkbar, um die Strömungseigenschaften des Kanals 66 positiv zu beeinflussen (nicht gezeigt).

Das Hinterteil 60 kann zudem mindestens eine Kanalwand 65 aufweisen. Die Kanalwände 65 sind in der Regel senkrecht zur Rückwand 63 angeordnet. Durch die Kanalwände 65 kann der Kanal 66 in mehrere Untereinheiten aufgeteilt sein.

Das Hinterteil 60 kann beliebig viele Kanalwände 65 enthalten, beispielsweise eine oder mehr, bevorzugt mindestens zwei. In einer konkreten Ausführungsform gemäß der Figuren kann das Hinterteil 60 beispielhaft drei Kanalwände 65 aufweisen, die den Kanal 66 in vier Untereinheiten aufteilen. Ausführungsformen mit mehr als drei Kanalwänden 65 sind auch möglich.

Die Kanalwände 65 haben eine Basis 65b und eine gegenüberliegende Stirnseite 65s (siehe Fig. 8). Die Kanalwände 65 verlaufen von ihrer Basis 65b zu ihrer Stirnseite 65s in der Regel gerade. Die Kanalwände 65 können mit ihrer Basis 65s auf der Rückwand 63 des Hinterteils angeordnet sein. Sodann können Kanalwände 65 und Rückwand 63 fest miteinander verbunden ausgebildet sein. Eine Verbindung der Kanalwände 65 mit der Rückwand ist nicht unbedingt nötig, verleiht den Kanalwänden allerdings eine zusätzliche Stabilität. Die Kanäle 63 sind zur Stirnseite 65s hin offen ausgebildet und können durch Auflage des Filtermaterials 70 zumindest bereichsweise abgedeckt werden.

Das Hinterteil 60 kann eine Auflagefläche 61 umfassen (siehe Fig. 9A). Die Auflagefläche 61 kann parallel zur Rückwand 63 verlaufen und ist beabstandet von dieser. Die Auflagefläche 61 kann eine dem Filtermaterial 70 entsprechende Außenkontur aufweisen, nämlich bevorzugt eine mehreckige Außenkontur, besonders bevorzugt eine viereckige. Beispielsweise ist die Außenkontur der Auflagefläche 61 trapezförmig ausgebildet. Das Filtermaterial 70 kann zur Herstellung eines Anwendungszustandes auf die Auflagefläche 61 und auf die Stirnseiten der Kanalwände 65s des Hinterteils 60 gelegt werden und sodann mit dem Vorderteil 50 verbunden werden. Die Kanalwände 65 können mit ihren Stirnseiten 65s somit insbesondere als zusätzliche Auflagemöglichkeit für das Filtermaterial 70 eingerichtet und ausgebildet sein.

Die Kanalwände 65 können in manchen Ausführungsformen auch andere Formen aufweisen. Beispielsweise können die Kanalwände 65 säulenförmig bzw. stelenförmig ausgebildet sein und dementsprechend nur punktuelle zusätzliche Auflagemöglichkeiten für das Filtermaterial 70 bereitstellen. Eine Unterteilung des Kanals 65 in Untereinheiten wäre sodann nicht gegeben (nicht gezeigt).

Die Auflagefläche 61 kann in manchen Ausführungsformen zusätzliche Elemente enthalten. Beispielsweise kann die Auflagefläche 61 zusätzliche Elemente aufweisen, die rippen- oder gitterförmig über den Kanal 66 gespannt sind, um zu verhindern, dass das Filtermaterial 70 in das Hinterteil 60 bzw. in den Kanal 66 hineingedrückt werden kann (nicht gezeigt).

Das Hinterteil 60 kann bevorzugt eine Kante 64 umfassen. Die Kante 64 steht senkrecht zur Auflagefläche 61 und kann die Auflagefläche 61 nach außen begrenzen. Das Filtermaterial 70 kann bevorzugt passgenau auf der Auflagefläche 61 angeordnet sein, wobei die Kante 64 das Filtermaterial 70 in Position halten kann. In einer bevorzugten Ausführungsform ist die Kante 64 dabei so hoch ausbildet, wie das Filtermaterial 70 dick ist.

Die Rückwand 63 und die Auflagefläche 61 bzw. die Stirnseiten der Kanalwände 65s sind derart voneinander beabstandet angeordnet und ausgebildet, dass der Luftraum bzw. die Kanäle 66 nach Auflage des Filtermaterials 70 ausgebildet werden (siehe Fig. 8). Die Seitenwand 62 kann dreiseitig umlaufend um den Kanal 66 ausgebildet sein.

In einer bevorzugten Ausführungsform ist das Hinterteil 60 im Wesentlichen trapezförmig. Insbesondere sind Auflagefläche 61 und Kante 64 trapezförmig ausgebildet. Hierbei kann die trapezförmige Auflagefläche 61 ein in der Regel ebenes Viereck mit vier Seiten 61-1, 61-2, 61-3 und 61-4 ausbilden. Die Auflagefläche 61 kann zwei parallel zueinander liegenden Grundseiten 61-1, 61-2 aufweisen. Die parallelen Grundseiten 61-1, 61-2 können unterschiedlich lang ausgebildet sein. Beispielsweise kann die Grundseite 61-1 länger ausgebildet sein als die Grundseite 61-2. Die beiden an die parallelen Grundseiten 61-1 und 61-2 angrenzenden Seiten des Auflagefläche 61-3, 61-4 können durch die unterschiedlich langen Grundseiten 61-1 und 61-2 nicht parallel verlaufen.

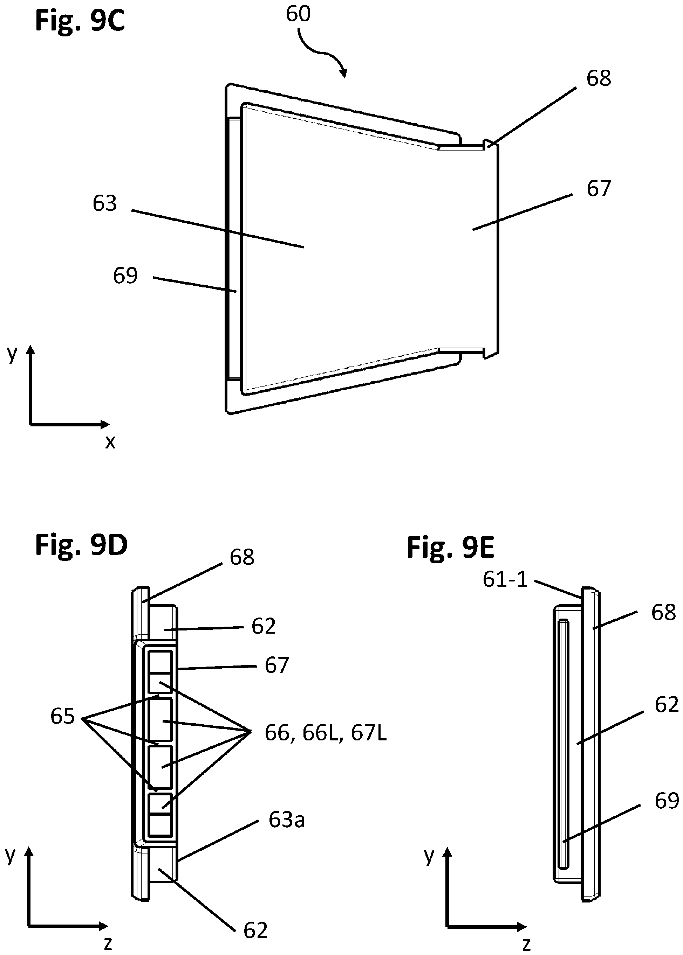

Der Kanal 66 oder die Kanäle 66 sind zu der zur kürzeren Grundseite 61-2 benachbart liegenden Seite hin offen und können dort an ein Tunnelelement 67 angrenzen (siehe Fig. 9). Das Tunnelelement 67 kann in einer beispielhaften Ausführungsform als aus der Trapezform herausragendes zusätzliches Element ausgebildet sein. Das Tunnelelement 67 kann benachbart zur Grundseite 61-2 der Auflagefläche sein. Das Tunnelelement 67 ist rohrförmig ausgebildet. Bevorzugt ist das Tunnelelement 67 als Vierkantrohr ausgebildet, das zu beiden Enden hin offen ausgebildet ist. Das Tunnelelement 67 weist ein Lumen 67L auf (siehe Fig. 9D). Das Lumen des Tunnelelements 67L und das Lumen der Kanäle 66L sind miteinander (Atem-)gasleitend verbunden.

Die Kanalwände 65 können in manchen Ausführungsformen das Lumen des Tunnelelements 67L durchlaufen, so dass das Lumen des Tunnelelements 67L entsprechend in Teillumen unterteilt sein kann.

Das Lumen des Tunnelelements 67L definiert einen hydraulischen Durchmesser der im Wesentlichen dem des Flügelkanals 15 entsprechend kann.

Bei Ausführungsformen mit Kanalwänden 65 definiert die Gesamtheit der Teillumen des Tunnelelements 67 einen hydraulischen Durchmesser der im Wesentlichen dem des Flügelkanals 15 entsprechend kann.

Das Lumen des Tunnelelements 67L kann einen hydraulischen Durchmesser von mindestens 10 mm aufweisen. In einer bevorzugten Ausführungsform weist das Lumen des Tunnelelements 67L einen hydraulischen Durchmesser von mindestens 12 mm auf. In einem konkreten Ausführungsbeispiel kann das Lumen des Tunnelelements 67L einen hydraulischen Durchmesser von 15 mm oder mehr aufweisen.

An das Tunnelelement 67 kann mindestens ein Arretierungsvorsprung 68 angeformt sein. Aus den Fig. 9A und 9B wird ersichtlich, dass der Arretierungsvorsprung 68 beispielsweise als dreiseitiger Vorsprung ausgebildet sein kann. Über den Arretierungsvorsprung 68 kann das Filterelement 40 nach Aufnahme in die Aufnahmeeinrichtung 16 des Maskenflügels 14 beispielsweise arretiert werden.

Alternativ oder zusätzlich zum Arretierungsvorsprung 68 kann das Hinterteil 60 und/oder das Vorderteil 50 eine Arretierungsschiene 69 aufweisen (siehe Fig. 9B, 9C, 9E). Über die Arretierungsschiene 69 kann das Filterelement 40 nach Aufnahme in die Aufnahmeeinrichtung 16 beispielsweise alternativ oder zusätzlich arretiert werden. Dazu kann die Aufnahmeeinrichtung 16, insbesondere die Aufnahme-Seitenwand 20, über entsprechende Arretierungsaussparungen 21 verfügen (siehe Fig. 12A). Die Arretierungsschiene 69 kann an der Seitenwand 62 des Hinterteils 60 angeformt sein. Hierbei ist es in manchen Ausführungen denkbar, dass die Arretierungsschiene 69 über die gesamte Länge der Seitenwand 62 verläuft (nicht gezeigt). In dem konkreten Ausführungsbeispiel gemäß der Figuren kann die Arretierungsschiene 69 lediglich an der Seitenwand 62 angeformt sein, die benachbart zur längeren Grundseite der Auflagefläche 61-1 liegt (siehe Fig. 9E). Die Arretierungsschiene 69 kann als langgestreckte Schiene gemäß der Figuren ausgebildet sein. In manchen Ausführungen kann die Arretierungsschiene 69 auch jegliche andere geeignete Form aufweisen. Beispielsweise ist es in einfacheren Ausführungsformen auch denkbar, dass die Arretierungsschiene 69 als punktueller Arretierungsvorsprung, beispielsweise Knopf- oder Hakenförmig ausgebildet ist.

Fig. 9D zeigt eine Seitenansicht des Hinterteils 60 mit Blick auf ein offenes Ende des Tunnelelements 67. Aus Fig. 9D wird ersichtlich, dass das Lumen des Kanals 66 L und/oder das Lumen des Tunnelelements 66L durch die Kanalwände 65 unterteilt sein kann. In diesem konkreten Ausführungsbeispiel ist der Kanal 66 und das Tunnelelement 67 in vier Teilabschnitte unterteilt.

Durch das Tunnelelement 67 kann der Luftaustausch stattfinden. In einem Anwendungszustand findet der Luftaustausch durch das Tunnelelement 67 und den Kanal 66 des Hinterteils 60 statt. Das offene Ende des Tunnelelements 67 zeigt in einem Anwendungszustand zum Flügelkanal 15. Kanäle 66, Tunnelelement 67 und Flügelkanal 15 sind sodann (Atem-)gasleitend miteinander verbunden.

In manchen Ausführungsformen kann der Kanal 66 im Filterelement 40 in Richtung vom Tunnelelement 67 zum Ende hin flacher werdend ausgebildet sein (nicht gezeigt). Dadurch kann eine Materialersparnis erreicht werden und die Atemmaske 1 insgesamt kann zierlicher ausgebildet sein.

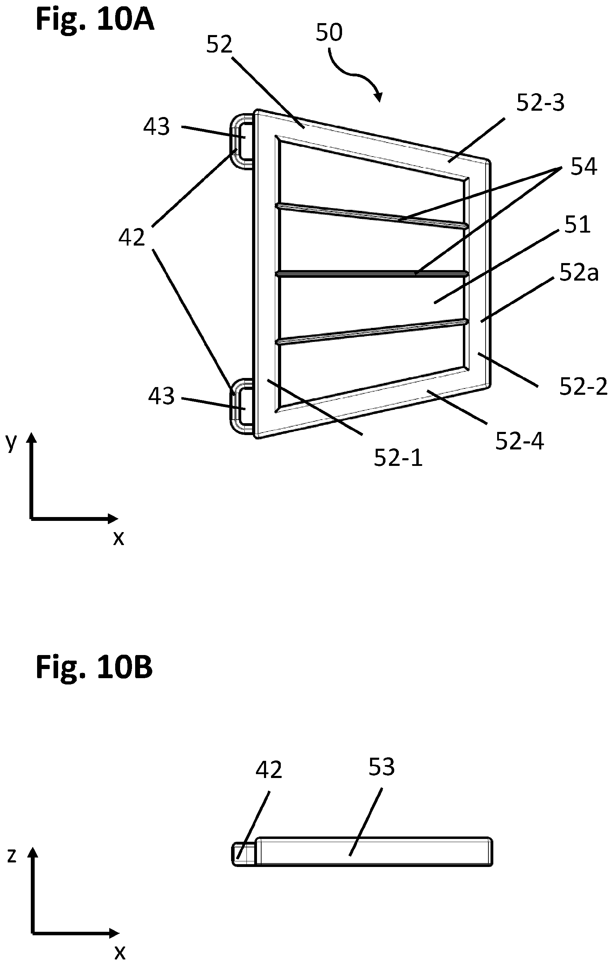

**Fig. 10 A-E** zeigen ein Vorderteil 50 eines Filterelements 40 aus unterschiedlichen Perspektiven. Die dargestellten Koordinatensysteme dienen der Verdeutlichung der Ausrichtung der Ansichten. Das Vorderteil 50 umfasst einen Rahmen 52.Der Rahmen 52 begrenzt das Vorderteil 40 nach außen. Der Rahmen 52 hat dementsprechend wie das Filterelement 40 insgesamt und wie das Hinterteil 60 eine mehreckige Form, bevorzugt eine viereckige. Besonders bevorzugt ist der Rahmen 52 trapezförmig ausgebildet.

In einer bevorzugten Ausführungsform ist der Rahmen 52 trapezförmig und bildet somit ein in der Regel ebenes Viereck mit vier Seiten 52-1, 52-2, 52-3 und 52-4. Der Rahmen 52 kann zwei parallel zueinander liegenden Grundseiten 52-1, 52-2 aufweisen. Die parallelen Grundseiten 52-1, 52-2 können unterschiedlich lang ausgebildet sein. Beispielsweise kann die Grundseite 52-1 länger ausgebildet sein als die Grundseite 52-2. Benachbart zur Grundseite 52-1 kann die mindestens eine Bänderungshalterung 42 angeformt sein. Bevorzugt sind benachbart zur Grundseite 52-1 zwei Bänderungshalterungen 42 angeformt. Bevorzugt liegen die beiden Bänderungshalterungen 42 maximal weit voneinander entfernt an den Enden der Grundseite 52-1. Die beiden an die parallelen Grundseiten 52-1 und 52-2 angrenzenden Seiten des Rahmens 52-3, 52-4 können durch die unterschiedlich langen Grundseiten 52-1 und 52-2 nicht parallel verlaufen.

Der Rahmen 52 mit seinen vier Seiten 52-1, 52-2, 52-3, 52-4 definiert eine Öffnung 51 in der inneren Fläche des Vorderteils 50, durch die der Luftaustausch stattfinden kann. In einem Anwendungszustand findet der Luftaustausch durch die Öffnung 51 des Vorderteil 50 und durch das Filtermaterial 70 statt.

Die Öffnung 51 kann ohne weitere Elemente ausgebildet sein (nicht gezeigt). In einer bevorzugten Ausführungsform kann innerhalb des Rahmens 52 und somit die Öffnung 51 durchlaufend mindestens eine Rippe 54 angeordnet sein. Die Rippen 54 können unter anderem dazu dienen, das Filtermaterial 70 zwischen dem Vorderteil 50 und dem Hinterteil 60 zu halten und gegen ein Herausrutschen zu sichern. Zudem kann dem Rahmen 52 durch die Rippen 54 eine zusätzliche Stabilität gegeben werden.

Die Rippen 54 können beliebig angeordnet sein und in einer beliebigen Anzahl ausgeführt sein. In manchen Ausführungsformen können die Rippen 54 auch fehlen (nicht gezeigt). In einer beispielhaften Ausführungsform gemäß den Figuren sind beispielsweise drei Rippen 54 beispielsweise nicht parallel zueinander angeordnet. Die Rippen 54 können auch in anderen Verläufen ausgeführt sein. Die Rippen 54 können in manchen Ausführungsformen auch parallel und/oder derart angeordnet sein, dass sie sich mittig in der vom Rahmen 52 definierten Öffnung 51 treffen bzw. kreuzen (nicht gezeigt).

Die Rippen 54 des Vorderteils 50 sind bevorzugt äquivalent zu den Kanalwänden 65 des Hinterteils 60 ausgebildet. Das bedeutet, dass die Kanalwände 65 und die Rippen 54 bevorzugt derart angeordnet sind, dass diese in einem Anwendungszustand des Filterelements 40 mit ihren Stirnseiten aufeinandertreffen. Dabei sind die die Stirnseiten 65s der Kanalwände 65 und die Stirnseiten der Rippen 54 nur durch das Filtermaterial 70 voneinander getrennt. Die Kanalwände 65 und die Rippen 54 können das Filtermaterial 70 zumindest bereichsweise einklemmen. Das Filtermaterial 70 kann somit in Position gehalten werden und ein Verrutschen des Filtermaterials 70 kann weitestgehend unterbunden werden.

Der Rahmen 52 weist eine Außenseite 52a und eine Innenseite 55i auf, wobei die Innenseite 52i in einem Anwendungszustand des Filterelements 40 zu dem Filtermaterial 70 weist. In Fig. 10A ist die Außenseite 52a dargestellt. Die Außenseite 52a kann glatt und/oder strukturiert ausgebildet sein. In einem einfachen Ausführungsbeispiel ist die Oberfläche der Außenseite 52a im Wesentlichen glatt ausgebildet. In manchen Ausführungsformen ist es denkbar, dass die Außenseite 52a eine strukturierte Oberfläche hat, um dem Anwender eine taktile Rückmeldung darüber zu geben, in welcher Richtung das Vorderteil 50 in das Filterelement 40 eingesetzt werden kann (nicht gezeigt).

Aus Fig. 10B wird ersichtlich, dass das Vorderteil 50 eine Seitenfläche 53 umfasst. Die Seitenfläche 53 ist am Rahmen 52 angeformt und verleiht dem Vorderteil 50 eine dreidimensionale Struktur. An die Seitenfläche 53 kann die mindestens eine Bänderungshalterung 42 angeformt sein.

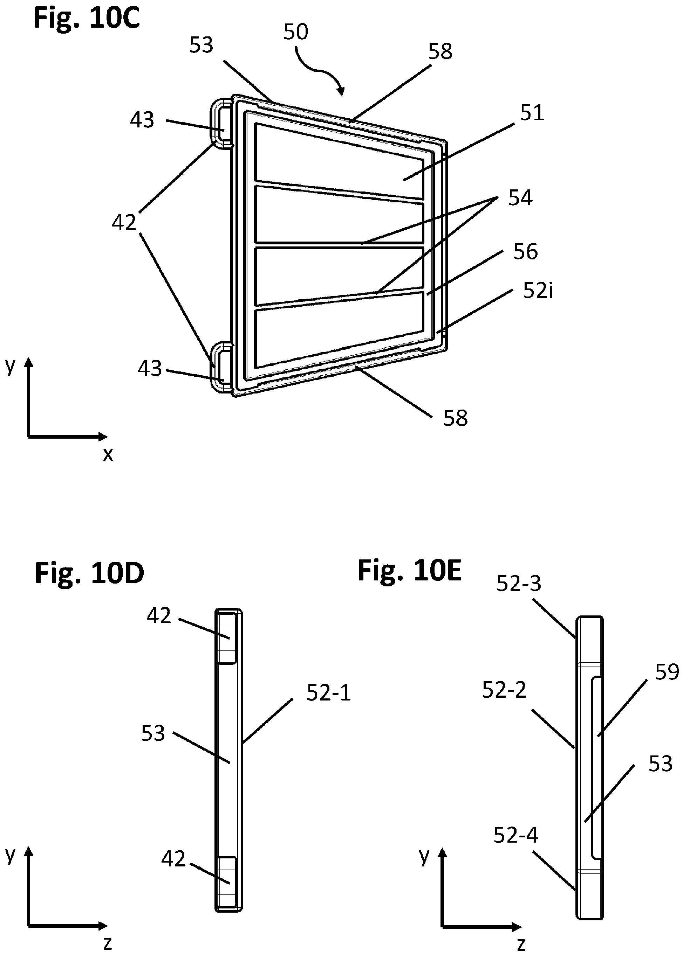

Aus Fig. 10D wird ersichtlich, dass die Bänderungshalterung 42 in diesem konkreten Ausführungsbeispiel in doppelter Ausführung vorhanden ist. Die zwei Bänderungshalterungen 42 können bevorzugt maximal weit voneinander entfernt an den Enden der Seitenfläche 53 angeformt sein, die benachbart zur Grundseite 52-1 liegen. In manchen Ausführungsformen können auch eine oder mehrere Bänderungshalterungen 42 umfasst sein. In Ausführungsform mit einer Bänderungshalterung 42 könnte eine Platzierung in der Mitte der Seitenfläche 53 vorteilhaft sein, um eine optimale Krafteinwirkung auf die Atemmaske 1 zu erwirken.

In Fig. 10C ist die Innenseite 52i dargestellt. Die Innenseite 52i kann eine Auflagefläche 56 aufweisen. Die Auflagefläche 56 kann im Wesentlichen umlaufend um die Öffnung 51 ausgebildet sein. Die Auflagefläche 56 kann gegenüber der Innenseite 52i erhaben ausgebildet sein. Beispielsweise sind die Auflagefläche 56 und die Stirnseite der Rippen 54 auf einem Höhenniveau und derart ausgebildet, dass das Filtermaterial 70 auf der Auflagefläche 56 und den Stirnseiten der Rippen 54 aufliegen kann. Insbesondere die Rippen 54 verhindern dabei, dass das Filtermaterial 70 durch den Rahmen 52 bzw. durch die Öffnung 51 aus der Atemmaske 1 herausfallen und/oder herausgedrückt werden kann.

An der Seitenfläche 53 kann mindestens eine Arretierung 58 angeformt sein. Über die Arretierung 58 kann das Vorderteil 50 mit dem Hinterteil 60 nach einer Verbindung mittels Einpressen verbunden sein. Sodann kann das Vorderteil 50 lediglich unter Aufwendung eines gewissen Druckes wieder von dem Hinterteil 60 getrennt werden.

Aus Fig. 10E wird ersichtlich, dass die Seitenfläche 53, die benachbart zur Grundseite 52-2 angeordnet ist, mindestens eine Aussparung 59 aufweisen kann. Die Aussparung 59 ist eingerichtet und ausgebildet, Bereiche des Hinterteils 60 zumindest teilweise zu umschließen.

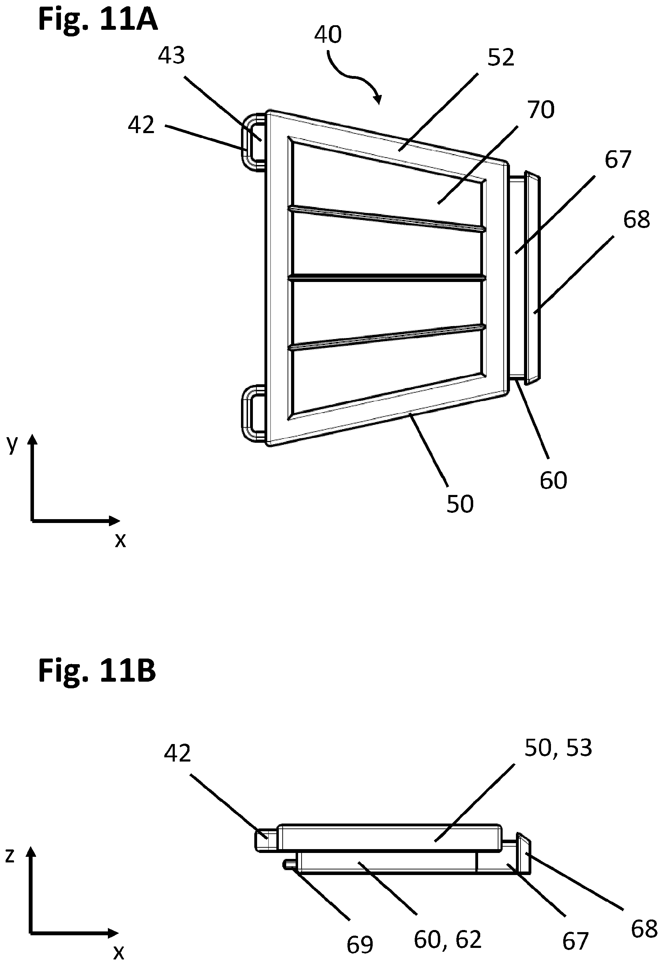

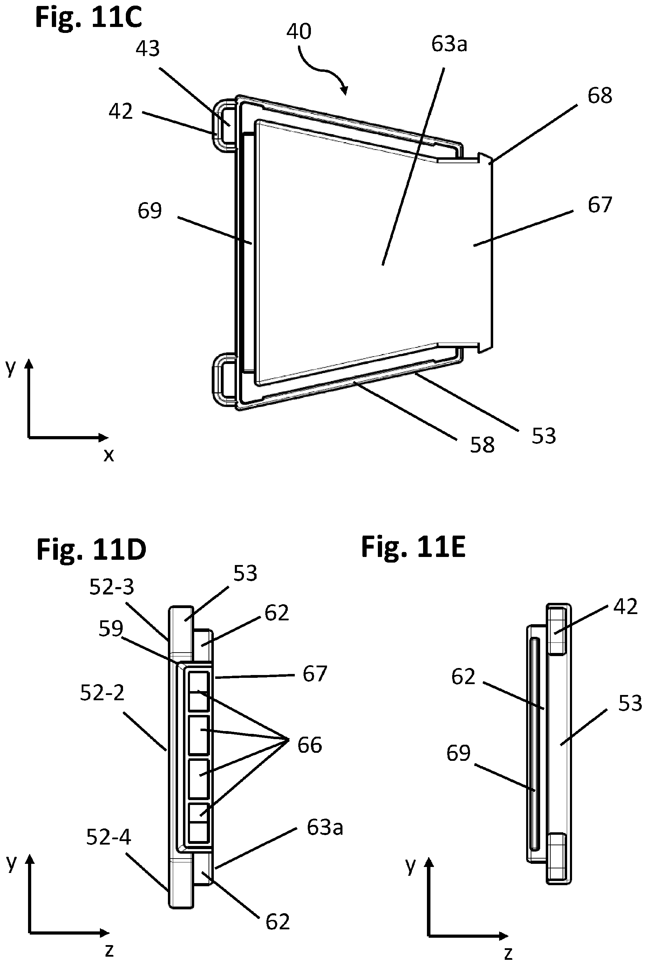

**Fig. 11 A-E** zeigen ein Filterelement 40 in einem Anwendungszustand aus unterschiedlichen Perspektiven. Im Anwendungszustand sind Vorderteil 50 und Hinterteil 60 des Filterelements 40 miteinander verbunden und umschließen ein Filtermaterial 70.

Aus Fig. 11A und 11B wird ersichtlich, dass Rahmen 52 und Seitenflächen 53 des Vorderteils 50 die Kante 64 des Hinterteils 60 umschließen und die Auflagefläche 61 bedecken. Insbesondere die Kante 64 des Hinterteils 60 ist dazu eingerichtet und ausgebildet, Vorderteil 50 und Hinterteil 60 miteinander zu verbinden. Zu diesem Zweck kann die Seitenfläche 53 des Vorderteils 50 beispielsweise derart über die Kante 64 des Hinterteils 60 gestülpt werden, dass Arretierung 58 das Vorderteil 50 in dieser Position arretiert (siehe auch Fig. 11C). Aus Fig. 11D wird ersichtlich, dass die Aussparung 59 in der Seitenfläche 53 eine Aufnahme des Tunnelelements 67 ermöglicht, ohne den Kanal 66 zu beeinflussen.

**Fig. 12** zeigt eine perspektivische Darstellung einer Atemmaske 1 mit Filterelementen 40 im Querschnitt und **Fig. 12** **A** eine Detailansicht aus Fig. 12. Fig. 12/12A zeigen beispielhaft, wie das Filterelement 40 im Maskenflügel 14 angeordnet ist.

Das Filterelement 40 befindet sich in einem Anwendungszustand, was bedeutet, dass Vorderteil 50 und Hinterteil 60 miteinander verbunden sind und ein Filtermaterial 70 umschließen. Hierbei ist die Kante 64 des Hinterteils 60 derart von Rahmen 52 und Seitenflächen 53 des Vorderteils 50 umschlossen, dass eine feste, jedoch in der Regel reversible Verbindung von Vorderteil 50 und Hinterteil 60 gegeben ist.

Das Filterelement 40 ist in der Aufnahmeeinrichtung 16 aufgenommen. Hierbei befindet sich die Rückwand 63 des Hinterteils 60 benachbart zur Aufnahme-Rückwand 18. Es wird ersichtlich, dass die Aufnahme-Rückwand 18 mit der Innenwand 15i des Flügelkanals 15 verbunden ist. Die Materialstärke von Aufnahme-Rückwand 18 und Innenwand 15i kann gleich oder unterschiedlich sein. In dem dargestellten konkreten Ausführungsbeispiel ist die Wandstärke der Aufnahme-Rückwand 18 geringer ausgebildet als die Wandstärke der Innenwand 15i. Über die geringere Wandstärke der Aufnahme-Rückwand 18 kann Material eingespart werden. In manchen Ausführungsformen kann die Aufnahme-Rückwand 18 auch gänzlich fehlen. Über die stärkere Wandstärke der Innenwand 15i kann der Flügelkanal 15 stabil ausgebildet sein und gegen übermäßige Verformungen geschützt sein. In manchen Ausführungsformen kann die Innenwand 15i Arretierungsaussparungen 21 aufweisen, in die Arretierungselemente des Filterelements 40 einrasten können, um das Filterelement 40 zusätzlich zu arretieren (nicht gezeigt).

Aus Fig. 12A wird ersichtlich, dass das Tunnelelement 67 nach Aufnahme des Filterelements 40 in die Aufnahmeeinrichtung 16 der Maskenflügel 14 zumindest bereichsweise innerhalb zumindest eines Teilbereiches des Flügelkanals 15 angeordnet ist. Auf diese Art stehen das Lumen des Flügelkanals 15L und das Lumen des Tunnelelements 67L miteinander fluidisch in Verbindung.

Durch die gestrichelten Pfeile ist eine (Atemgas-) Strömung S schematisch angedeutet. Die Strömung S stellt den Atemgasstrom des Anwenders nach Anlage der Atemmaske 1 dar. Die Strömung S kann in beide Richtungen strömen.

Bei einer Inspiration kann die Atemgasströmung S durch das Filtermaterial 70 in den mindestens einen Kanal 66 strömen. Die Strömung S gelangt sodann vom Kanal 66 in das Tunnelelement 67 und vom Tunnelelement 67 in den Flügelkanal 15 und vom Flügelkanal 15 in Richtung Mund und/oder Nase des Anwenders (nicht gezeigt). Bei einer Exspiration nimmt die Atemgasströmung S die entgegengesetzte Richtung. Die Strömung S kann insbesondere durch das Lumen des Flügelkanals 15L und das Lumen des Tunnelelements 67L und das Lumen des Kanals 66L strömen. Vom Lumen des Kanals 66L verläuft die Strömung durch das Filtermaterial 70 in die Umgebungsluft ab und vice versa.

Im gezeigten konkreten Ausführungsbeispiel sind die Kanäle des Tunnelelements 67 und die Kanäle 66 konstant in Höhe und Breite ausgebildet. In manchen Ausführungsbeispielen ist es denkbar, dass der Kanal 67 und/oder der Kanal 66 sich verjüngend und/oder aufweitend ausgebildet ist. Beispielsweise kann der Kanal 66 derart eingerichtet und ausgebildet sein, dass er sich in Richtung zum Flügelkanal 15 hin trichterförmig aufweitet. Eine solche trichterförmige Aufweitung kann den Strömungsweg positiv beeinflussen (nicht gezeigt).

Das konkrete Ausführungsbeispiel des Filterelements 40 gemäß der Figuren weist einen Arretierungsvorsprung 68 und eine Arretierungsschiene 69 auf. Über den Arretierungsvorsprung 68 und die Arretierungsschiene 69 ist das Filterelement 40 innerhalb der Aufnahmeeinrichtung 16 bzw. des Maskenflügels 14 arretiert. Arretierungsvorsprung 68 und Arretierungsschiene 69 sind hierfür jeweils in entsprechenden Arretierungsaussparungen 21 der Aufnahme-Seitenwand 20 bzw. der Außenwand 15a des Flügelkanals 15 aufgenommen. Arretierungsaussparungen 21 in der Innenwand 15i und/oder der Oberwand 15o und/oder der Unterwand 15u des Flügelkanals 15 sind ebenfalls denkbar (nicht gezeigt).

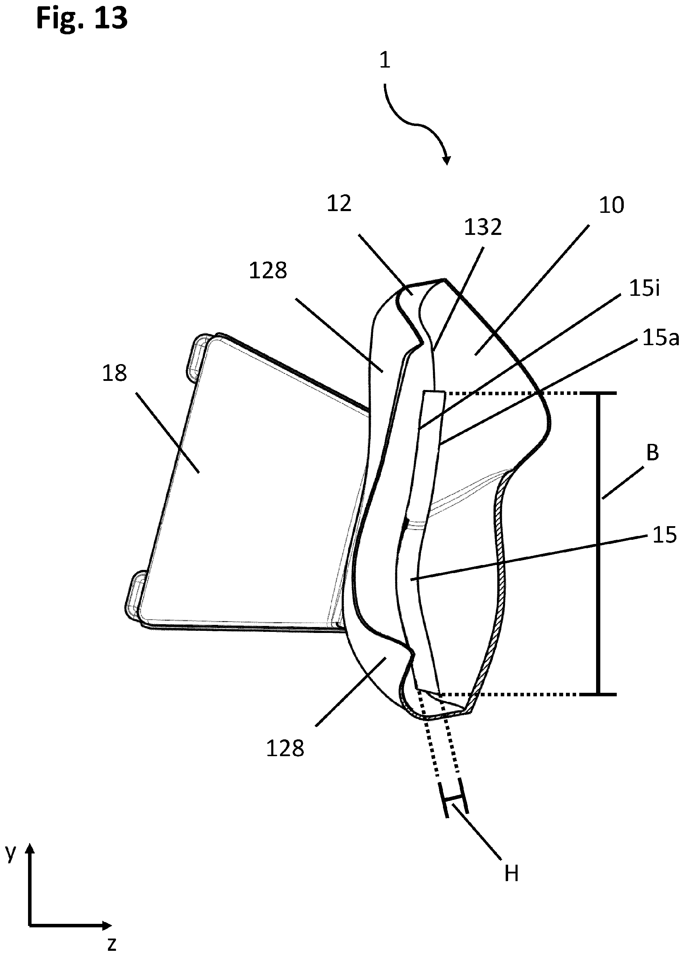

**Fig. 13** zeigt eine Darstellung einer Atemmaske 1 im Querschnitt. Der Flügelkanal 15 kann eine Höhe H und eine Breite B aufweisen. Die Breite B des Flügelkanals 15 ist in der Regel größer als die Höhe H des Flügelkanals 15.

Aus der Querschnittsdarstellung der Fig. 13 wird ersichtlich, dass der Flügelkanal 15 derart angeordnet sein kann, dass der Flügelkanal 15 zumindest abschnittsweise zwischen Maskenkörper 10 und Maskenwulst 12 angeordnet ist. Der Flügelkanal 15 kann beispielsweise derart zwischen Maskenkörper 10 und Maskenwulst 12 angeordnet sein, dass die Außenwand 15a des Flügelkanals 15 über die Breite B mit dem Maskenkörper 10 verbunden ist und die Innenwand 15 i über die Breite B mit dem Maskenwulst 12. In manchen Ausführungsformen ist denkbar, dass der Flügelkanal 15 im oder am Maskenkörper 10 angeordnet ist. In alternativen Ausführungsformen ist es auch denkbar, dass der Flügelkanal 15 im oder am Maskenwulst 12 angeordnet ist (nicht gezeigt).

Die Breite B des Flügelkanals 15 kann in einem Bereich zwischen 30 mm und 100 mm liegen, bevorzugt in einem Bereich zwischen 50 mm und 80 mm. In einem konkreten Ausführungsbeispiel beträgt die Breite B des Flügelkanals 15 72 mm.

Die Höhe H des Flügelkanals 15 kann in einem Bereich zwischen 4 mm und 12 mm liegen, bevorzugt in einem Bereich zwischen 5 mm und 8 mm. In einem konkreten Ausführungsbeispiel beträgt die Höhe H des Flügelkanals 15 6,5 mm.

Obwohl die vorliegende Erfindung anhand der Ausführungsbeispiele detailliert beschrieben wurde, ist es für den Fachmann selbstverständlich, dass die Erfindung nicht auf diese Ausführungsbeispiele beschränkt ist. Vielmehr sind Abwandlungen in einer Weise möglich, dass einzelne Merkmale weggelassen werden oder andersartige Kombinationen der beschriebenen Einzelmerkmale verwirklicht werden können, sofern der Schutzbereich der beiliegenden Ansprüche nicht verlassen wird. Die vorliegende Offenbarung schließt sämtliche Kombinationen der vorgestellten Einzelmerkmale mit ein.

### Bezugszeichenliste

- 1: Atemmaske
- 2: Atemgasraum
- 10: Maskenkörper
- 12: Maskenwulst
- 14: Maskenflügel
- 15: Flügelkanal
- 15a: Außenwand
- 15i: Innenwand
- 15o: Oberwand
- 15u: Unterwand
- 15L: Lumen des Flügelkanals
- 16: Aufnahmeeinrichtung
- 18: Aufnahme-Rückwand
- 20: Aufnahme-Seitenwand
- 21: Arretierungsaussparungen
- 24: Nasenbereich
- 28: Mundbereich
- 30: Anschlussbereich
- 32: Aufnahmeöffnung
- 40: Filterelement
- 42: Bänderungshalterung
- 43: Durchführungsöffnung
- 50: Vorderteil
- 51: Öffnung
- 52: Rahmen
- 52-1, 52-2, 52-3, 52-4: Seiten des Rahmens
- 52a: Außenseite des Rahmens
- 52i: Innenseite des Rahmens
- 53: Seitenfläche
- 54: Rippe
- 56: Auflagefläche
- 58: Arretierung
- 59: Aussparung
- 60: Hinterteil
- 61: Auflagefläche
- 61-1, 61-2, 61-3, 61-4: Seiten der Auflagefläche
- 62: Seitenwand
- 63: Rückwand
- 63a: Rückwand Außenseite
- 63i: Rückwand Innenseite
- 64: Kante
- 65: Kanalwand
- 65b: Basis der Kanalwand
- 65s: Stirnseite der Kanalwand
- 66: Kanal
- 66L: Lumen des Kanals
- 67: Tunnelelement
- 67L: Lumen des Tunnelelements
- 68: Arretierungsvorsprung
- 69: Arretierungsschiene
- 70: Filtermaterial
- 90: Kopf
- 121: Eckpunkt
- 122: Eckpunkt
- 123: Eckpunkt
- 124: Eckpunkt
- 125: Nasenrückenbereich
- 126: Seitenbereich
- 127: Basisbereich
- 128: Auflagebereich
- 129: Einkerbung
- 130: Dichtlippe
- 131: Kante der Dichtlippe
- 132: Übergang

- A: Richtung zum Anwender
- B: Breite
- E: Symmetrieebene
- E1: Ebene
- E3: Ebene
- E5: Ebene
- E7: Ebene
- H: Höhe
- S: Strömung
- α: Winkel
- β: Winkel

## Patentansprüche

1. Atemmaske 1 umfassend einen Maskenkörper 10, einen Maskenwulst 12, zumindest einen Maskenflügel 14 und zumindest ein Filterelement 40, wobei der Maskenwulst 12 mit dem Maskenkörper 10 verbunden ist, wobei der Maskenflügel 14 mit dem Maskenkörper 10 und/oder dem Maskenwulst 12 verbunden ist, wobei der Maskenwulst 12 zumindest abschnittsweise zur Anlage an der Haut eines Anwenders ausgebildet ist, wobei der Maskenwulst 12 bei Anwendung der Atemmaske 1 derart an der Haut des Anwenders anliegt, dass die Atemmaske 1 im Wesentlichen atemgasdicht abschließt, wobei der Maskenwulst 12 eine Aufnahmeöffnung 32 aufweist, die ausgebildet und eingerichtet ist, bei Anwendung der Atemmaske 1 zumindest Nase und Mund des Anwenders aufzunehmen **dadurch gekennzeichnet, dass** das Filterelement 40 mit dem Maskenflügel 14 verbunden ist, wobei das Filterelement 40 derart eingerichtet und ausgebildet ist, dass es zumindest bereichsweise von Atemgas durchströmbar ist.

2. Atemmaske 1 nach Anspruch 1 **dadurch gekennzeichnet, dass** die Atemmaske 1 mindestens zwei Maskenflügel 14 umfasst, die mit jeweils mindestens einem Filterelement 40 verbunden sind, wobei die Verbindung von Maskenflügeln 14 und Filterelementen 40 reversibel oder irreversibel ist.

3. Atemmaske 1 nach einem der voranstehenden Ansprüche **dadurch gekennzeichnet, dass** das Filterelement 40 ein Filtermaterial 70 ist und/oder ein Filtermaterial 70 umfasst, wobei das Filtermaterial 70 anorganische Partikel und/oder organische Partikel, insbesondere Keime, aus dem Atemgas filtert und zurückhält, wobei das Filtermaterial 70 austauschbar ist.

4. Atemmaske 1 nach einem der voranstehenden Ansprüche **dadurch gekennzeichnet, dass** das Filtermaterial 70 eine Fläche von mindestens 5 cm² aufweist, bevorzugt mindestens 20 cm², insbesondere mindestens 35 cm².

5. Atemmaske 1 nach einem der voranstehenden Ansprüche **dadurch gekennzeichnet, dass** der Maskenkörper 10, der Maskenwulst 12 und die Maskenflügel 14 mit den Filterelementen 40 bei Anwendung der Atemmaske 1 einen Atemgasraum 2 definieren, in dem sich Mund und Nase des Anwenders befinden, wobei eine Atemgasströmung vom Mund und Nase des Anwenders weg sowie zum Mund und Nase des Anwenders hin ausschließlich durch das Filtermaterial 70 möglich ist.

6. Atemmaske 1 nach einem der voranstehenden Ansprüche **dadurch gekennzeichnet, dass** der Atemgasraum 2 ein Volumen von maximal 500 ml, bevorzugt maximal 200 ml, insbesondere maximal 120 ml aufweist.

7. Atemmaske 1 nach einem der voranstehenden Ansprüche **dadurch gekennzeichnet, dass** die Atemmaske 1 aus einem elastomeren und/oder einem formstabilen Kunststoff gefertigt ist, bevorzugt ausgewählt aus der Gruppe Polycarbonate (PC), Polystyrole (PS), Polyamide (PA), Polypropylene (PP), Polyoxymethylene (POM), Polyacrylnitrile (PAN), Thermoplastische Elastomere (TPE), Acrylnitril-Styrol-Acrylester (ASA), Acrylnitril-Butadien-Styrole (ABS), Styrol-Acrylnitrile (SAN), Styrol-Methyl-Methacrylate (SMMA), Polyethylene (PE), Cycloolefin-Copolymere (COC), Polytetrafluorethylene (PTFE), Silikone, wobei die Atemmaske 1 desinfizierbar und/oder sterilisierbar und/oder autoklavierbar ist.

8. Atemmaske 1 nach einem der voranstehenden Ansprüche **dadurch gekennzeichnet, dass** der Maskenkörper 10 und/oder der Maskenwulst 12 und/oder die Maskenflügel 14 mehrstückig oder einstückig gefertigt sind, bevorzugt einstückig.

9. Atemmaske 1 nach einem der voranstehenden Ansprüche **dadurch gekennzeichnet, dass** der Maskenkörper 10 und/oder der Maskenwulst 12 und/oder die Maskenflügel 14 im Wesentlichen aus einem elastomeren Kunststoff gefertigt sind, bevorzugt aus Silikon.

10. Atemmaske 1 nach einem der voranstehenden Ansprüche **dadurch gekennzeichnet, dass** das Silikon bevorzugt klarsichtig ist, wobei das Silikon eine Lichtdurchlässigkeit von mindestens 50% aufweist, bevorzugt von mindestens 80%, besonders bevorzugt von mindestens 90%.

11. Atemmaske 1 nach einem der voranstehenden Ansprüche **dadurch gekennzeichnet, dass** der Maskenkörper 10 und/oder der Maskenwulst 12 und/oder die Maskenflügel 14 eine Shore-Härte von 30 bis 60 Shore A aufweisen, wobei der Maskenkörper 10 und/oder der Maskenwulst 12 und/oder die Maskenflügel 14 eine maximale Dehnbarkeit zwischen 100% und 800% aufweisen.

12. Atemmaske 1 nach einem der voranstehenden Ansprüche **dadurch gekennzeichnet, dass** die Atemmaske 1 mindestens eine Bänderungshalterung 42 umfasst, die eingerichtet ist, zumindest ein Kopfbefestigungsband aufzunehmen, wobei die Bänderungshalterungen 42 an den Maskenflügeln 14 und/oder an den Filterelementen 40 angeordnet sind, bevorzugt an den Filterelementen 40.

13. Atemmaske 1 nach einem der voranstehenden Ansprüche **dadurch gekennzeichnet, dass** die Maskenflügel 14 mindestens eine Aufnahmeeinrichtung 16 zur Aufnahme des Filterelementes 40 umfassen, wobei die Aufnahmeeinrichtung 16 weitestgehend formkomplementär zum Filterelement 40 ausgebildet ist, wobei die Aufnahmeeinrichtung 16 und das Filterelement 40 im Wesentlichen eine mehreckige Grundform aufweisen, bevorzugt eine viereckige Grundform, besonders bevorzugt eine rautenförmige oder trapezförmige Grundform.

14. Atemmaske 1 nach einem der voranstehenden Ansprüche **dadurch gekennzeichnet, dass** die Aufnahmeeinrichtung 16 mindestens eine Aufnahme-Seitenwand 20 umfasst, die elastisch verformbar ist, wobei das Filterelement 40 nach Aufnahme in die Aufnahmeeinrichtung 16 von der Aufnahme-Seitenwand 20 zumindest teilweise umgeben ist und durch diese in der Aufnahmeeinrichtung 16 fixiert wird.

15. Atemmaske 1 nach einem der voranstehenden Ansprüche **dadurch gekennzeichnet, dass** die Maskenflügel 14 mindestens einen Flügelkanal 15 mit einem Lumen 15L umfassen und dass das Filterelement 40 mindestens einen Kanal 66 mit einem Lumen 66L umfasst und dass das Filterelement 40 ein Tunnelelement 67 mit einem Lumen 67L aufweist, wobei das Tunnelelement 67 zumindest teilweise in dem Flügelkanal 15 der Maskenflügel 14 angeordnet ist, wobei die Lumina 15L, 67L, 66L zumindest teilweise den Atemgasraum 2 definieren.

16. Atemmaske 1 nach einem der voranstehenden Ansprüche **dadurch gekennzeichnet, dass** das Filterelement 40 ein Vorderteil 50 und ein Hinterteil 60 mit einer Auflagefläche 61 und das Filtermaterial 70 umfasst, wobei das Filtermaterial 70 zwischen dem Vorderteil 50 und dem Hinterteil 60 angeordnet ist, wobei das Filtermaterial 70 derart auf der Auflagefläche 61 platziert ist, dass der Kanal 66 zumindest bereichsweise durch das Filtermaterial 70 begrenzt ist.

17. Atemmaske 1 nach einem der voranstehenden Ansprüche **dadurch gekennzeichnet, dass** das Hinterteil 60 mindestens eine Kanalwand 65 mit einer Basis 65b und einer Stirnseite 65s umfasst, wobei die Kanalwand 65 den Kanal 66 in Untereinheiten unterteilt.

18. Atemmaske 1 nach einem der voranstehenden Ansprüche **dadurch gekennzeichnet, dass** das Vorderteil 50 eine Öffnung 51 umfasst, durch die ein Luftaustausch zwischen dem Atemgasraum 2 und der Umgebungsluft stattfindet, wobei das Filtermaterial 70 derart zwischen dem Atemgasraum 2 und der Öffnung 51 platziert ist, dass der Luftaustausch ausschließlich durch das Filtermaterial 70 stattfindet.

19. Atemmaske 1 nach einem der voranstehenden Ansprüche **dadurch gekennzeichnet, dass** der Flügelkanal 15, das Tunnelelement 67 und der Kanal 66 gasleitend miteinander verbunden sind, wobei das Atemgas vom Mund und/oder Nase des Anwenders über das Lumen 15L in das Lumen 67L und vom Lumen 67L in das Lumen 66L und vom Lumen 66L ausschließlich durch das Filtermaterial 70 strömt und vice versa.

20. Atemmaske 1 nach einem der voranstehenden Ansprüche **dadurch gekennzeichnet, dass** der Flügelkanal 15 und/oder das Tunnelelement 67 einen hydraulischen Durchmesser von mindestens 10 mm aufweisen, bevorzugt mindestens 12 mm, besonders bevorzugt von mindestens 15 mm.

21. Filterelement 40 für eine Atemmaske 1 nach einem der voranstehenden Ansprüche.
